# FASCICULE DE BREVET EUROPEEN

(11) **EP 3 071 692 B1**
(45) Date de publication et mention de la délivrance du brevet: **02.05.2018**
(21) Numéro de dépôt: 14821723.5
(22) Date de dépôt: 21.11.2014
(51) Int. Cl.: C12N 9/42, C12N 15/56

(54) **VARIANTS D'ENDOGLUCANASES A ACTIVITE AMELIOREE ET LEURS UTILISATIONS**
ENDOGLUCANASE-VARIANTEN MIT VERBESSERTER WIRKUNG UND VERWENDUNGEN DAVON
ENDOGLUCANASE VARIANTS HAVING IMPROVED ACTIVITY, AND USES OF SAME

(30) Priorité: 22.11.2013 FR 1361509
(43) Date de publication de la demande: 28.09.2016
(73) Titulaire: IFP Energies nouvelles, 92500 Rueil-Malmaison (FR); Proteus, 91160 Longjumeau (FR); Centre National de la Recherche Scientifique (C.N.R.S.), 75794 Paris Cedex 16 (FR)
(72) Inventeur: MARGEOT, Antoine, F-75018 Paris (FR); BENOIT, Yves, F-95480 Pierrelaye (FR); PERSILLON, Cécile, F-30000 Nimes (FR); AYRINHAC, Céline, F-30350 Domessargues (FR); ULLMANN, Christophe, F-30000 Nimes (FR); BONZOM, Olivier, F-30000 Nimes (FR); FORT, Sébastien, F-38410 Vaulnaveys-le-Haut (FR); ARMAND, Sylvie, 38000 Grenoble (FR); PETIT, Maud, F-59700 Marcq en Baroeul (FR); LENON, Marine, F-38360 Sassenage (FR)
(74) Mandataire: Cabinet Plasseraud
(86) Numéro de dépôt international: PCT/FR2014/052984
(87) Numéro de publication internationale: WO 2015/075391

(56) Documents cités:
- WO-A1-2008/148131
- WO-A1-2010/088387
- WO-A1-2011/057140
- WO-A1-2011/059740
- WO-A1-2013/028701
- WO-A2-2009/085935
- WO-A2-2011/153516
- WO-A2-2012/036810
- WO-A2-2012/044915
- GUNSELI BAYRAM AKCAPINAR ET AL: "From in silico to in vitro: Modelling and production of Trichoderma reesei endoglucanase 1 and its mutant in Pichia pastoris", JOURNAL OF BIOTECHNOLOGY, vol. 159, no. 1-2, 1 mai 2012 (2012-05-01) , pages 61-68, XP055039736, ISSN: 0168-1656, DOI: 10.1016/j.jbiotec.2012.01.001

## Description

La possibilité de produire de l'éthanol à partir de la cellulose a reçu beaucoup d'attention en raison de la disponibilité de grandes quantités de matière première ainsi que de l'intérêt de l'éthanol à titre de carburant. Les matières premières naturelles cellulosiques pour un tel processus sont désignées par le terme "biomasse". De nombreux types de biomasse, par exemple le bois, les résidus agricoles, les cultures herbacées et les déchets solides municipaux, ont été considérés comme des matières premières potentielles pour la production de biocarburant. Ces matières sont constituées principalement de cellulose, d'hémicellulose et de lignine.

La cellulose est un polymère constitué de molécules de glucose reliées par des liens beta 1-4, qui sont très résistants à la dégradation ou à la dépolymérisation. Une fois la cellulose convertie en glucose, celui-ci est facilement fermenté en biocarburant, par exemple l'éthanol, en utilisant une levure.

Les plus anciennes méthodes étudiées pour convertir la cellulose en glucose sont basées sur l'hydrolyse acide. Ce processus peut se faire en présence d'acides concentrés ou dilués. Cependant, plusieurs inconvénients tels que la mauvaise récupération de l'acide lors de l'utilisation d'acides concentrés et la faible production de glucose dans le cadre de l'utilisation d'acides dilués nuisent à l'économie du processus d'hydrolyse acide.

Pour surmonter les inconvénients du processus d'hydrolyse acide, les processus de conversion de la cellulose ont porté plus récemment sur l'hydrolyse enzymatique, à l'aide d'enzymes de type cellulase. Cette hydrolyse enzymatique de la biomasse lignocellulosique (par exemple, la cellulose) présente cependant l'inconvénient d'être un procédé industriel coûteux. De ce fait, il est nécessaire d'utiliser des souches de microorganismes sécréteurs de cellulases de plus en plus performantes. À ce titre, beaucoup de microorganismes comportent des enzymes qui hydrolysent la cellulose, tels que les champignons *Trichoderma, Aspergillus, Humicola, Fusarium* ainsi que des bactéries telles que *Thermomonospora, Bacillus, Cellulomonas* et *Streptomyces.* Les enzymes sécrétées par ces microorganismes possèdent trois types d'activités utiles dans la conversion de la cellulose en glucose et se divisent en trois groupes: les endoglucanases, qui attaquent les fibres de celluloses aléatoirement en interne, les exoglucanases qui vont attaquer les extrémités des fibres en libérant du cellobiose, et les β-glucosidases qui vont hydrolyser ce cellobiose en glucose. D'autres classes d'enzymes telles que les hémicellulases ou la classe d'enzymes récemment découverte des polysaccharides mono-oxygénases peuvent jouer également un rôle dans l'efficacité de l'hydrolyse.

Il y a un intérêt industriel fort pour la diminution du coût de l'hydrolyse enzymatique, et cette diminution passe par l'utilisation d'une dose réduite d'enzymes et donc des cocktails d'enzymes plus efficaces. En conséquence, plusieurs demandes de brevets décrivent des enzymes naturelles aux capacités supérieures à celles de *Trichoderma reesei,* ou des variants améliorés par génie génétique. On peut citer les demandes de brevet US2010304464, WO2010066411 et WO2013029176 concernant les exoglucanases, les demandes WO2007109441, WO2012149192, WO2012036810 et WO2010076388 concernant les endoglucanases, les demandes WO2010029259, WO2010135836, WO2012044915, WO2011057140 ou WO2010022518 concernant les beta-glucosidases, ou encore les demandes WO12135659, WO12149344 concernant les polysaccharides mono-oxygénases.

La demande WO2011153516 décrit également des souches de levures exprimant des enzymes saccharolytiques.

La demande WO2013028701 décrit également des variants de glycoside hydrolase GHG61.

La demande WO2008141131 décrit des protéines ayant une activité cellulolytique améliorée.

Gunseli Bayram Akcapinar et al. décrivent la production d'endonucléase I de Trichoderma reesei dans Journal of Biotechnology, vol. 159, n°1-2, 1 mai 2012, pages 61-68.

Les enzymes hydrolysant la biomasse lignocellulosique sont classées dans le système CAZy (Cantarel, B. L., Coutinho, P. M., Rancurel, C., Bernard, T., Lombard, V., & Henrissat, B. (2009). The Carbohydrate-Active EnZymes database (CAZy): an expert resource for Glycogenomics. Nucleic acids research, 37, D233-8) sur des critères principalement structuraux. Les endoglucanases peuvent appartenir aux familles GH 5, 6, 7, 8, 9, 12, 16, 18, 19, 26, 44, 45, 48, 51, 74 et 124.

Pour qu'une hydrolyse de la biomasse lignocellulosique soit efficace et économiquement rentable, le mélange enzymatique doit comporter des proportions équilibrées d'enzymes ayant des activités enzymatiques diverses, entre autres, mais non exclusivement, du type exoglucanases, endoglucanases, xylanases et β-glucosidases. A titre d'exemple, dans les mélanges natifs de *Trichoderma reesei* on constate généralement la présence de 60-70% d'exoglucanases, 15-20% d'endoglucanases, quelques pourcentages d'hémicellulases et environ 5-10% de β-glucosidases. Ce mélange convient pour hydrolyser la majorité des substrats prétraités (ex. type paille de blé explosée à la vapeur en conditions acides) avec des rendements acceptables. En somme, l'augmentation de l'activité endoglucanase ne doit pas se faire au détriment des autres activités enzymatiques. Les spécificités fonctionnelles de ces enzymes sont aujourd'hui mal connues. Le génome de *Trichoderma reesei* comporte au moins 3 enzymes principales, issues des familles 7 (EG1, cel7b), 5 (EG2, cel5a) et 12 (EG3, Cel12a). Les enzymes EG1 et EG2 sont les endoglucanases majoritaires et peuvent représenter jusqu'à 10-20% en masse du cocktail d'enzymes complet produit par *T. reesei.*

Les endoglucanases (EC 3.2.1.4), premières enzymes à agir sur la cellulose, sont connues pour avoir un rôle majeur dans l'hydrolyse en augmentant le nombre de sites que peuvent attaquer les exoglucanases tout en diminuant le degré de polymérisation des microfibrilles attaquées. De récents travaux (Szijártó, N., Siika-aho, M., Sontag-Strohm, T., & Viikari, L. (2011). Liquefaction of hydrothermally pretreated wheat straw at high-solids content by purified Trichoderma enzymes. Bioresource technology, 102(2), 1968-74) soulignent leur rôle dans la diminution de la viscosité de la biomasse au cours des premières heures de l'hydrolyse. Cette diminution de viscosité peut avoir un impact très important sur les coûts opératoires du procédé.

Le problème de viscosité est exacerbé dans le cas de procédés imposant le recours à une basse température telle que la saccharification simultanée à la fermentation (SSF), qui met en jeu à la fois les enzymes hydrolysant la biomasse et le micro-organisme qui convertit les monomères de sucres en éthanol.

L'hydrolyse et la fermentation peuvent être réalisées suivant différents schémas. Le plus courant consiste en une hydrolyse et une fermentation séparées (SHF - Separate Hydrolysis and Fermentation). Cette méthode permet d'optimiser chaque étape par le maintien des conditions optimales de réaction. Cette fermentation s'effectue de manière extemporanée, à une température comprise entre environ 28°C et environ 30°C, tandis que l'hydrolyse a lieu généralement à une température d'au moins 45°C. Cependant, en SHF, les sucres libérés en fin de réaction sont présents à très forte concentration et entraînent une inhibition des enzymes, ralentissant l'efficacité du procédé. Pour éviter ces inconvénients, un autre type de procédé peut être envisagé. En SSF, les deux étapes (hydrolyse et fermentation des hexoses) ont lieu de manière simultanée, empêchant l'accumulation des sucres à des concentrations inhibitrices pour les enzymes. Les coûts d'investissement sont également réduits grâce à l'utilisation d'un seul réacteur. Le taux d'hydrolyse est plus élevé suite à l'absence d'inhibition car les sucres libérés sont utilisés immédiatement pour la fermentation en éthanol. Dans cette méthode, la température du réacteur constitue nécessairement un compromis entre les températures optimales d'hydrolyse et de fermentation, typiquement entre environ 30°C et environ 35°C. Cependant, à une telle température, l'activité des enzymes cellulolytiques est diminuée de 30% environ.

La SSF permet également l'expression d'enzymes dégradant la cellulose dans l'organisme fermentant les sucres, ce qui permet de limiter, ou dans un cas extrême de supprimer le recours aux enzymes produites lors d'une étape séparée. Cependant, produire de grandes quantités d'enzymes avec les organismes fermentaires et donc obtenir une activité importante peut s'avérer problématique et limite la viabilité de ces approches.

En conséquence, l'obtention d'enzymes maintenant une activité endoglucanase efficace aux températures optimales d'hydrolyse et de fermentation (soit entre 30°C et 50°C) tout en gardant la proportion de l'ensemble des enzymes du mélange serait un gain significatif pour le procédé de conversion de biomasse lignocellulosique en biocarburant.

### Description

Les inventeurs ont développé un polypeptide ayant une activité endoglucanase améliorée, notamment par rapport à l'activité endoglucanase de la protéine sauvage EG1 de séquence SEQ ID NO :2. EG1 correspond à l'endoglucanase 1 de *Trichoderma reesei.*

Dans cette optique, les déposants, ont eu le grand mérite de trouver, après de nombreuses recherches, un polypeptide isolé ou purifié ayant une activité endoglucanase améliorée par rapport à l'activité endoglucanase de la protéine de référence EG1 (SEQ ID NO :2).

La description concerne donc un polypeptide choisi dans le groupe consistant en :
i) une séquence d'acides aminés choisie parmi SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24 et SEQ ID NO :26;
ii) une séquence d'acides aminés présentant un pourcentage d'identité d'au moins 70%, préférentiellement 75%, 80%, 85%, 90%, 95%, 98% ou 99%, par rapport à la séquence SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24 ou SEQ ID NO :26.

L'invention est définie dans les revendications.

De préférence, le polypeptide selon l'invention est caractérisé en ce que son expression dans un organisme fermentaire est au moins égale à l'expression de la protéine de référence EG1 (SEQ ID NO : 2).

Le pourcentage d'identité d'une séquence donnée par rapport à SEQ ID NO :4, 6, 8, 10, 12, 14, 16, 18, 20, 22, 24 ou 26, correspond au nombre de résidus identiques entre cette séquence donnée et SEQ ID NO :4, 6, 8, 10, 12, 14, 16, 18, 20, 22, 24 ou 26 divisé par le nombre de résidus dans SEQ ID NO :4, 6, 8, 10, 12, 14, 16, 18, 20, 22, 24 ou 26. Lorsqu'on utilise la base de données GenomeQuest, ledit pourcentage d'identité par rapport à SEQ ID NO : 4, 6, 8, 10, 12, 14, 16, 18, 20, 22, 24 ou 26 correspond au pourcentage d'identité d'Interrogation (% id Query), où Interrogation correspond à la séquence SEQ ID NO :4, 6, 8, 10, 12, 14, 16, 18, 20, 22, 24 ou 26.

L'homme du métier pourra par exemple déterminer l'augmentation ou autrement dit l'amélioration de l'activité enzymatique soit à l'aide du substrat Carboxy-Méthyl-Cellulose (CMC), soit avec un substrat chromogénique (p-Nitrophenyl glycoside). L'activité enzymatique sera respectivement révélée par dosage colorimétrique des sucres réducteurs ou bien du nitrophénol libérés.

De préférence, le polypeptide de l'invention a une activité enzymatique améliorée d'au moins 10%, préférentiellement d'au moins 20%, préférentiellement d'au moins 30%, par rapport à l'activité endoglucanase de la protéine EG1 de séquence d'acides aminés SEQ ID NO :2.

Un exemple de protocole, que l'homme du métier pourra utiliser pour déterminer si un polypeptide selon l'invention présente une activité enzymatique améliorée par rapport à celle de la protéine de référence EG1 (SEQ ID NO :2), est le suivant :
- formation d'une culture stock d'*E. coli* exprimant un polypeptide selon l'invention pendant toute la nuit à 37°C;
- ensemencement d'un milieu de culture LB avec 1% de culture stock à 37°C jusqu'à l'obtention d'une densité optique de 0.4 ;
- culture desdites cellules à 20°C pendant 18h ;
- centrifugation pendant 5 minutes à 7900 rpm ;
- resuspension des culots cellulaires avec du tampon citrate phosphate 100 mM à pH 5 contenant 1 mg/mL de lysozyme (DO₆₀₀ finale 100);
- incubation des cellules resuspendues 30 minutes sur la glace ;
- lyse des cellules par 3 cycles de congélation/décongélation ;
- fractionnement de l'ADN par sonication ;
- centrifugation 30 minutes à 13000 rpm ;
- incubation de 100 µL de surnageant de cassage avec 100 µL de tampon citrate phosphate 100 mM à pH 5 contenant 1% de CMC pendant 6h à 35 et 50°C ;
- prélèvement de 100 µL de réaction ;
- ajout de 100 µL de réactif DNS (Miller, 1959);
- incubation 5 minutes à 100°C ;
- incubation 3 minutes sur la glace ;
- centrifugation pendant 10 minutes à 3000 rpm ;
- lecture de la densité optique à 540 nm sur 150 µL de surnageant.

L'invention a également pour objet un acide nucléique purifié ou isolé codant au moins un polypeptide selon l'invention tel que décrit précédemment. Le TABLEAU 1 ci-dessous comprend les identifications des séquences nucléiques et peptidiques pour EG1 de T. reesei ("sauvage"), les endoglucanases putatives de *Chaetomium globosum* (C) et d'*Aspergillus fumigatus* (A), ainsi que pour les polypeptides et nucléotides de l'invention et de la description.

Selon la description, ledit acide nucléique purifié ou isolé peut être choisi parmi les séquences suivantes: SEQ ID NO :3, SEQ ID NO :5, SEQ ID NO :7, SEQ ID NO :9, SEQ ID NO :11; SEQ ID NO :13, SEQ ID NO :15, SEQ ID NO :17, SEQ ID NO :19, SEQ ID NO :21, SEQ ID NO :23 et SEQ ID NO :25.

**TABLEAU 1**

| **Clones** | **Acide nucléique** | **Polypeptide** |
|---|---|---|
| EG1 (sauvage) | SEQ ID NO :1 | SEQ ID NO :2 |
| 76B4 | SEQ ID NO :3 | SEQ ID NO :4 |
| 105F11 | SEQ ID NO :5 | SEQ ID NO :6 |
| 107H12 | SEQ ID NO :7 | SEQ ID NO :8 |
| 154E4 | SEQ ID NO :9 | SEQ ID NO :10 |
| 202C12 | SEQ ID NO :11 | SEQ ID NO :12 |
| 272A9 | SEQ ID NO :13 | SEQ ID NO :14 |
| 278F10 | SEQ ID NO :15 | SEQ ID NO :16 |
| 293B2 | SEQ ID NO :17 | SEQ ID NO :18 |
| 309A11 | SEQ ID NO :19 | SEQ ID NO :20 |
| 11G8 | SEQ ID NO :21 | SEQ ID NO :22 |
| 92A12 | SEQ ID NO :23 | SEQ ID NO :24 |
| 240H12 | SEQ ID NO :25 | SEQ ID NO :26 |
| Gène C | SEQ ID NO :27 | SEQ ID NO :28 |
| Gène A | SEQ ID NO :29 | SEQ ID NO :30 |

L'invention porte également sur un vecteur comprenant un acide nucléique selon l'invention tel que décrit précédemment.

Selon l'invention, on entend par « vecteur » toute séquence d'ADN dans laquelle il est possible d'insérer des fragments d'acide nucléique étranger, les vecteurs permettant d'introduire de l'ADN étranger dans une cellule hôte. On peut citer de manière non-exhaustive comme vecteurs : les plasmides, les cosmides, les chromosomes artificiels de levures (YAC), les chromosomes artificiels de bactéries (BAC), les chromosomes artificiels dérivés du bactériophage P1 (PAC) ou les vecteurs dérivés de virus.

Selon l'invention, l'acide nucléique tel que décrit précédemment pourra être lié opérationnellement à un promoteur, un terminateur ou toute autre séquence nécessaire à son expression dans la cellule hôte.

Le vecteur selon l'invention pourra également porter un marqueur de sélection. On entend par « marqueur de sélection » un gène dont l'expression confère aux cellules qui le contiennent une caractéristique permettant de les sélectionner. Il s'agit par exemple d'un gène de résistance aux antibiotiques.

L'invention a également pour objet une cellule hôte isolée comprenant soit au moins l'un des polypeptides selon l'invention tels que décrit précédemment, soit au moins l'un des acides nucléiques selon l'invention tels que décrit précédemment ou soit au moins l'un des vecteurs selon l'invention tels que décrit précédemment.

L'homme du métier pourra introduire l'un des polypeptides, l'un des acides nucléiques ou l'un des vecteurs selon l'invention tels que décrit précédemment dans la cellule hôte selon l'invention par des méthodes conventionnelles bien connues. Par exemple, on peut citer le traitement au chlorure de calcium, l'électroporation, l'utilisation d'un pistolet à particules.

Selon un mode de réalisation, l'homme du métier pourra introduire dans la cellule hôte selon l'invention et par des méthodes conventionnelles, plusieurs copies d'un acide nucléique selon l'invention codant un polypeptide selon l'invention ayant une activité endoglucanase améliorée selon l'invention.

Selon un mode de réalisation, la cellule hôte isolée selon l'invention telle que décrite précédemment est choisie parmi *Trichoderma, Aspergillus, Neurospora, Humicola, Myceliophthora, Chrysosporium, Pénicillium, Fusarium, Thermomonospora, Bacillus, Pseudomonas, Escherichia, Clostridium, Cellulomonas, Streptomyces, Yarrowia, Pichia* et *Saccharomyces.*

Selon un mode de réalisation préféré, la cellule hôte isolée selon l'invention telle que décrite précédemment est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Myceliophthora thermopila, Chrysosporium lucknowense, Neurospora crassa, Humicola grisae, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* et *Saccharomyces cerevisiae.*

Selon un mode de réalisation préféré, la cellule hôte isolée selon l'invention telle que décrite précédemment est choisie parmi *Trichoderma reesei* et *Saccharomyces cerevisiae.*

L'invention a également pour objet l'utilisation de l'un quelconque des polypeptides selon l'invention décrits précédemment pour l'hydrolyse de la cellulose.

L'invention a également pour objet l'utilisation de l'un quelconque des polypeptides selon l'invention décrits précédemment pour la production de biocarburant.

Selon l'invention, le terme biocarburant peut être défini comme étant tout produit issu de la transformation de la biomasse et pouvant être utilisé à des fins énergétiques. D'une part et sans vouloir se limiter, on peut citer à titre d'exemple des biogaz, des produits pouvant être incorporés (éventuellement après transformation ultérieure) à un carburant ou être un carburant à part entière, tels que des alcools (l'éthanol, le butanol et/ou l'isopropanol selon le type d'organisme fermentaire utilisé), des solvants (acétone), des acides (butyrique), des lipides et leurs dérivés (acides gras à courtes ou longues chaînes, esters d'acides gras), ainsi que l'hydrogène.

De manière préférée, le biocarburant selon l'invention est un alcool, par exemple l'éthanol, le butanol et/ou l'isopropanol. Plus préférentiellement, le biocarburant selon l'invention est l'éthanol.

Dans un autre mode de réalisation, le biocarburant est du biogaz.

Dans un autre mode de réalisation, le produit est une molécule intéressant l'industrie chimique, comme par exemple, un autre alcool tel que le 1,2-propane diol, le 1,3-propane diol, le 1,4-butane diol, le 2,3-butane diol, des acides organiques comme l'acide acétique, propionique, acrylique, butyrique, succinique, malique, fumarique, citrique, itaconique, ou des hydroxyacides comme l'acide glycolique, hydroxypropionique, ou lactique.

On décrit ci-dessous un mode de réalisation de production d'un cocktail enzymatique utile pour l'hydrolyse de la lignocellulose.

Les souches de champignons filamenteux, de préférence *Trichoderma,* plus préférentiellement *T. reesei,* capables d'exprimer au moins un polypeptide selon l'invention sont cultivées en fermenteurs, en présence d'un substrat carboné, tel que le lactose ou le glucose, choisi pour la croissance du microorganisme. Dans un mode de réalisation, ce substrat carboné, selon sa nature, est introduit dans le fermenteur avant stérilisation ou est stérilisé séparément et introduit dans le fermenteur après stérilisation de ce dernier pour obtenir une concentration initiale de 20 à 35 g/L.

Une solution aqueuse contenant le substrat choisi pour la production des enzymes est ensuite ajoutée. Une composition enzymatique agissant sur la biomasse lignocellulosique produite par les champignons est enfin récupérée par filtration du milieu de culture. Dans cette composition, on retrouve notamment, la β-glucosidase, l'exoglucanase et l'endoglucanase selon l'invention.

Dans un mode de réalisation, la solution aqueuse contenant le substrat choisi pour la production des enzymes est préparée à la concentration de 200-250 g/L. Cette solution contient en outre de préférence un substrat inducteur tel que le lactose. Cette solution aqueuse est injectée après l'épuisement du substrat carboné initial de façon à apporter une quantité optimisée, comprise entre 35 et 45 mg/g de cellules ("fed batch"). Pendant cette phase de "fed batch", la concentration résiduelle en sucre dans le milieu de culture est inférieure à 1 g/L et les enzymes agissant sur la biomasse lignocellulosique sont sécrétées par le champignon. Ces dernières peuvent être récupérées par filtration du milieu de culture.

L'invention a pour objet une composition enzymatique apte à agir sur la biomasse lignocellulosique, ladite composition enzymatique étant produite par des champignons filamenteux et comprenant au moins un polypeptide ayant une activité endoglucanase améliorée par rapport à l'activité endoglucanase de la protéine de référence EG1. Par « champignons filamenteux », on entend notamment *Trichoderma,* plus préférentiellement *T. reesei.*

Enfin, l'invention a pour objet un procédé de production de biocarburant à partir de la biomasse comprenant les étapes successives suivantes :
- mise en suspension en phase aqueuse de la biomasse à hydrolyser;
- hydrolyse en présence d'une composition enzymatique de la biomasse lignocellulosique telle que décrite précédemment de manière à produire un hydrolysat contenant du glucose;
- fermentation du glucose de l'hydrolysat de manière à produire un moût de fermentation;
- séparation du biocarburant du moût de fermentation.

Dans un mode de réalisation, la biomasse à hydrolyser est mise en suspension en phase aqueuse à raison de 6 à 40% de matière sèche, de préférence 20 à 30%. Le pH est ajusté entre 4 et 5,5; de préférence entre 4,8 et 5,2 et la température entre 40 et 60°C, de préférence entre 45 et 50°C. La réaction d'hydrolyse est démarrée par l'ajout de la composition enzymatique agissant sur la biomasse lignocellulosique; la quantité habituellement utilisée est de 10 à 30 mg de protéines excrétées par gramme de substrat prétraité ou moins. La réaction dure généralement de 15 à 48 heures. La réaction est suivie par dosage des sucres libérés, notamment le glucose. La solution de sucres est séparée de la fraction solide non hydrolysée, essentiellement constituée de lignine, par filtration ou centrifugation et ensuite traitée dans une unité de fermentation.

Après l'étape de fermentation, le biocarburant est séparé du moût de fermentation par exemple par distillation.

Un autre objet de l'invention est un procédé de production de biocarburant à partir de la biomasse, caractérisé en ce qu'il comprend les étapes successives suivantes :
- mise en suspension en phase aqueuse de la biomasse à hydrolyser;
- ajout simultané d'une composition enzymatique telle que définie précédemment et d'un organisme fermentaire de manière à produire un moût de fermentation;
- séparation du biocarburant du moût de fermentation.

De préférence, la composition enzymatique et l'organisme fermentaire sont ajoutés simultanément puis incubés à une température comprise entre 30°C et 35°C pour produire un moût de fermentation.

Selon ce mode de réalisation, la cellulose présente dans la biomasse est convertie en glucose, et en même temps, dans le même réacteur, l'organisme fermentaire (par exemple une levure) convertit le glucose en produit final selon un procédé de SSF (Simultaneous Saccharification and Fermentation) connu de l'homme du métier. Selon les capacités métaboliques et hydrolytiques de l'organisme fermentaire, le bon déroulement de l'opération peut nécessiter l'addition d'une quantité plus ou moins importante de mélange cellulolytique exogène.

Dans un autre mode de réalisation, l'organisme fermentaire produit le polypeptide objet de l'invention par sécrétion ou en surface de sa cellule, éventuellement conjointement à d'autres enzymes agissant sur la biomasse lignocellulosique, limitant ou supprimant ainsi le besoin en enzymes produites par le champignon filamenteux. De préférence, l'organisme fermentaire est une cellule hôte telle que décrite précédemment.

Ainsi, de préférence, l'invention a pour objet un procédé de production de biocarburant à partir de la biomasse, comprenant les étapes successives suivantes :
- mise en suspension en phase aqueuse de la biomasse à hydrolyser ;
- ajout d'une ou plusieurs cellules hôtes telles que décrites précédemment, avec un organisme fermentaire et/ou une composition enzymatique telle que décrite précédemment, de manière à produire un moût de fermentation ;
- séparation du biocarburant du moût de fermentation.

De préférence, les cellules hôtes avec la composition enzymatique et/ou l'organisme fermentaire, sont ajoutées puis incubés à une température comprise entre 30°C et 35°C pour produire un moût de fermentation.

L'utilisation du polypeptide présentant une meilleure activité endoglucanase selon la présente invention présente ainsi l'avantage d'obtenir un meilleur rendement de production de glucose tout en employant moins d'enzyme qu'auparavant, ce qui présente également un avantage économique.

D'autres aspects, objets, avantages et caractéristiques de l'invention, seront présentés à la lecture de la description non restrictive qui suit et qui décrit des modes de réalisation préférés de l'invention donnés par le biais d'exemples et des FIGURES.

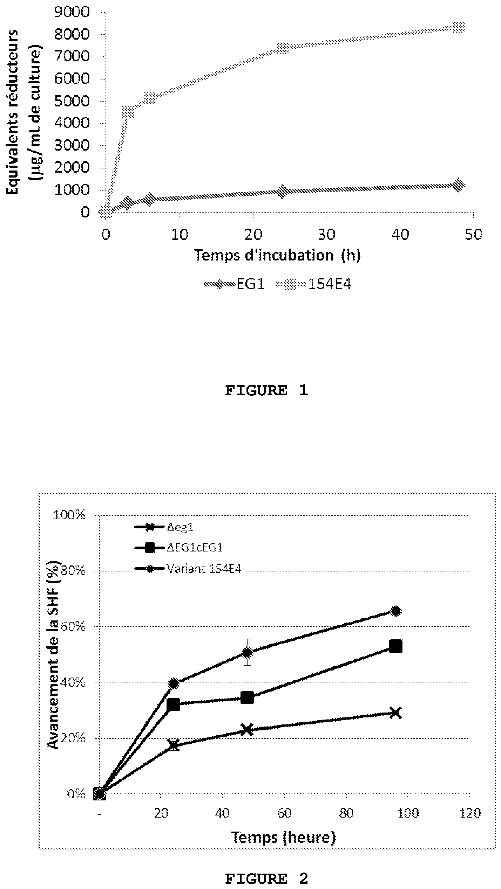

La FIGURE 1 est un graphique représentant l'hydrolyse de la CMC 1% par l'endoglucanase de référence (EG1) et son mutant (154E4) sécrétés dans le milieu de culture par les souches Scα-EG1 et Scα-154E4 respectivement.

La FIGURE 2 est un graphique présentant les résultats de SHF pour le cocktail issu de la souche 154E4/8 (SEQ ID NO :10), un cocktail de référence produit par la souche CL847 ΔEG1 (ΔEG1) supplémenté en β-glucosidase et un autre cocktail de référence produit par la souche CL847 ΔEG1 retransformée avec le gène de référence EG1 (ΔEG1cEG1) supplémenté en β-glucosidase.

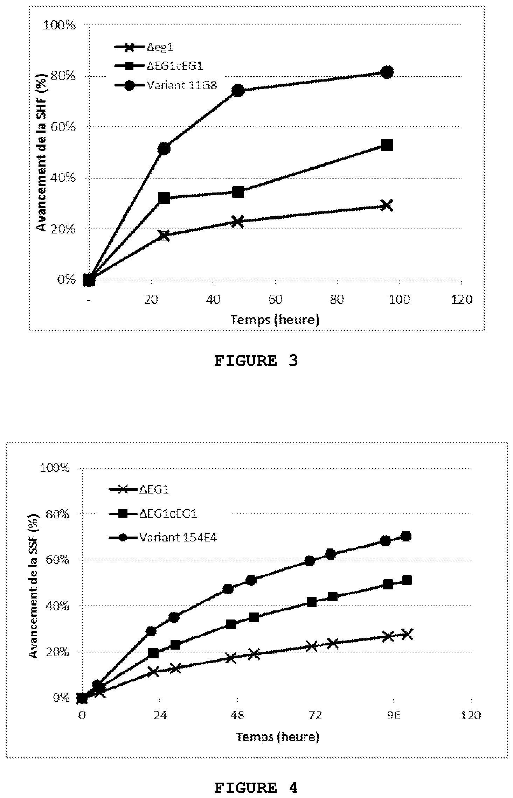

La FIGURE 3 est un graphique présentant les résultats de SHF pour le cocktail issu de la souche 11G8/10 (SEQ IQ NO :22), un cocktail de référence produit par la souche CL847 ΔEG1 (ΔEG1) supplémenté en β-glucosidase et un autre cocktail de référence produit par la souche CL847 ΔEG1 retransformée avec le gène de référence EG1 (ΔEG1cEG1) supplémenté en β-glucosidase.

La FIGURE 4 est un graphique présentant les résultats de SSF pour les deux cocktails issus des souches 154E4/2 154 E4/8 (SEQ ID NO :10), un cocktail de référence produit par la souche CL847 ΔEG1 (ΔEG1) supplémenté en β-glucosidase et un autre cocktail de référence produit par la souche CL847 ΔEG1 retransformée avec le gène de référence EG1 (ΔEG1cEG1) supplémenté en β-glucosidase.

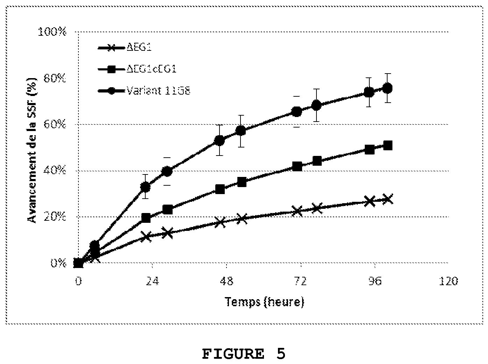

La FIGURE 5 est un graphique présentant les résultats de SSF pour les trois cocktails issus des souches 11G8/10, 11G8/12 et 11G8/13 (SEQ ID NO :22), un cocktail de référence produit par la souche CL847 ΔEG1 (ΔEG1) supplémenté en β-glucosidase et un autre cocktail de référence produit par la souche CL847 ΔEG1 retransformée avec le gène de référence EG1 (ΔEG1cEG1) supplémenté en β-glucosidase.

### EXEMPLES

### EXEMPLE 1 : 1er tour de L-shuffling

La séquence du gène de référence EG1 de *Trichoderma reesei* (SEQ ID NO :1) a été soumise à un premier tour de L-shuffling selon le procédé décrit dans EP1104457B1 avec les gènes de l'endoglucanase putative de *Chaetomium globosum* (SEQ ID NO :29) et de l'endoglucanase d'*Aspergillus fumigatus* (SEQ ID NO :27) présentant chacune environ 60% d'identité avec le gène de référence SEQ ID NO :1.

### 1-Criblage à haut débit

Un test de criblage à haut débit a été mis au point afin de sélectionner les meilleurs clones issus du L-shuffling, c'est-à-dire ceux présentant au moins 20% d'amélioration de l'activité endoglucanase par rapport à l'enzyme de référence SEQ ID NO :2.

Le test de criblage à haut débit a été réalisé selon les étapes suivantes :
- isolement sur gélose des clones de *E. coli* exprimant les variants de L-shuffling de l'enzyme recombinante selon l'invention et mise en pré-cultures en milieu LB desdites colonies pendant la nuit à 37°C ;
- inoculation d'un milieu LB à 6% avec la pré-culture puis incubation 5 heures à 37°C puis 17 heures à 20°C ;
- centrifugation 10 minutes à 3000 rpm ;
- lyse des cellules par ajout de 80 µL d'une solution de lysozyme à 1 mg/mL dans du tampon citrate phosphate 0,1 M à pH 5 ;
- incubation pendant 4 heures à température ambiante;
- ajout de 80 µL de tampon citrate phosphate 0,1 M pH 5 contenant 1% de carboxy-méthyl cellulose ;
- incubation 17 heures à 35°C ;
- centrifugation pendant 10 minutes à 3000 rpm ;
- prélèvement de 100 µL de surnageant ;
- ajout de 100 µL de réactif DNS ;
- incubation 10 minutes à 100°C puis 5 minutes sur la glace ;
- lecture de la DO à 540 nm sur 120 µL.

Dans ces conditions de criblage haut débit, une amélioration de l'activité endoglucanase (augmentation de la DO à 540 nm) par rapport à l'enzyme de référence EG1 (SEQ ID NO :2) a été trouvée dans plusieurs clones, dont notamment les clones 76B4,105F11, 107H12, 154E4, 202C12, 272A9, 278F10, 293B2 et 309A11.

### 2-Détermination de l'amélioration de l'activité endoglucanase

### 2-1/ Sur le substrat carboxy-méthyl cellulose (CMC)

Afin de d'estimer le kcat des variants sélectionnés au premier tour de L-shuffling par rapport à l'enzyme de référence (SEQ ID NO :2), on procède de la façon suivante :
- préparation d'une culture stock d'*E. coli* exprimant une enzyme recombinante selon l'invention pendant toute la nuit à 37°C ;
- ensemencement d'un milieu de culture LB avec 1% de culture stock à 37°C jusqu'à l'obtention d'une densité optique à 600 nm de 0.4 ;
- culture desdites cellules à 20°C pendant 18 heures;
- centrifugation pendant cinq minutes à 7900 rpm ;
- resuspension des culots cellulaires avec du tampon citrate phosphate 0.1M à pH 5 contenant 1 mg/mL de lysozyme (DO₆₀₀ finale 100);
- incubation des cellules resuspendues 30 minutes sur la glace ;
- lyse des cellules par 3 cycles de congélation/décongélation ;
- fractionnement de l'ADN par sonication pendant 3 secondes à puissance 5 ;
- centrifugation 30 minutes à 13000 rpm ;
- incubation de 100 µL de surnageant de cassage avec 100 µL de tampon citrate phosphate 0.1 M à pH 5 contenant 1% de CMC pendant 6 heures à 35 et 50°C ;
- prélèvement de 100 µL de réaction ;
- ajout de 100 µL de réactif DNS ;
- incubation 5 minutes à 100°C ;
- incubation 3 minutes sur la glace ;
- centrifugation 10 minutes à 3000 rpm ;
- lecture de la densité optique à 540 nm sur 150 µL.

Selon l'invention, le calcul des kcat est fait de la façon suivante :
- expression des DO à 540 nm en fonction de la quantité de protéine d'intérêt (en nM) ;
- soustraction de la valeur du témoin négatif ;
- division par le coefficient de la gamme étalon de glucose (différentes quantités de glucose sont révélées avec le DNS) ;
- division par le temps de réaction (360 minutes).

Le TABLEAU 2 présente la valeur des kcat ainsi que le facteur d'amélioration obtenus pour les clones 76B4, 105F11, 107H12, 154E4, 202C12, 236C11, 272A9, 278F10, 293B2 et 309A11 par rapport à la protéine de référence EG1 (SEQ ID NO :2) dans les conditions expérimentales du test d'activité sur CMC.

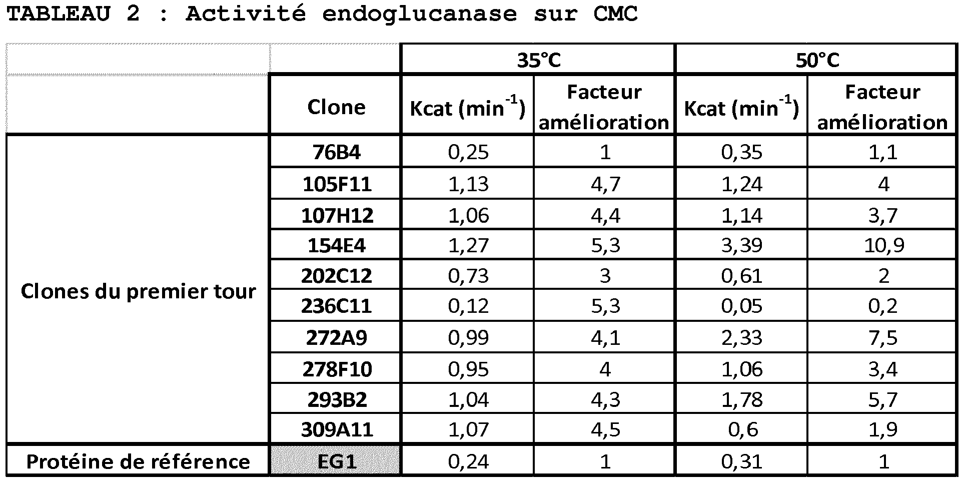

Les résultats montrent, pour ces clones, une amélioration d'activité enzymatique par rapport à l'enzyme de référence SEQ ID NO :2.

### 2-2/ Sur le substrat phosphoric acid swollen cellulose (PASC)

L'amélioration d'activité des clones 76B4, 105F11, 107H12, 154E4, 202C12, 236C11, 272A9, 278F10, 293B2 et 309A11 a ensuite été confirmée sur un second substrat : phosphoric acid swollen cellulose (PASC).

La détermination du kcat sur ce substrat est effectuée selon le même protocole que décrit précédemment. Le substrat CMC est remplacé par le substrat PASC à la même concentration.

Le TABLEAU 3 présente la valeur des kcat ainsi que le facteur d'amélioration obtenus pour les clones 76B4, 105F11, 107H12, 154E4, 202C12, 236C11, 272A9, 278F10, 293B2 et 309A11 par rapport à la protéine de référence EG1 (SEQ ID NO :2) dans les conditions expérimentales du test d'activité sur PASC.

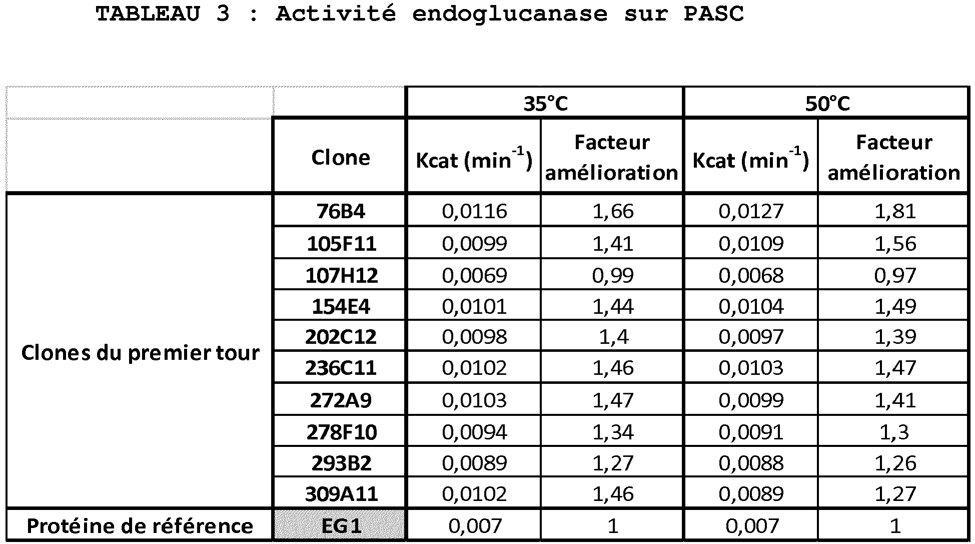

Ces résultats montrent une amélioration de l'activité enzymatique par rapport à l'enzyme de référence EG1 (SEQ ID NO :2).

### EXEMPLE 2 : 2ème tour de L-shuffling

Les gènes améliorés 105F11, 154E4, 202C12, 272A9, 278F10 et 309A10 (respectivement SEQ ID NO :5, 9, 11, 13, 15, 19) obtenus au premier tour d'évolution ont par la suite été soumis à un deuxième tour de L-shuffling (toujours selon le procédé breveté décrit dans EP1104457B1). Afin de favoriser la reconstruction sur un squelette de la séquence de *Trichoderma,* le gène de référence EG1 (SEQ ID NO :1) a été réintroduit en tant que gène parent pour le deuxième tour de L-shuffling.

### 1-Criblage à haut débit

Un test de criblage à haut débit tel que décrit précédemment a été effectué sur les clones obtenus à la suite de ce second tour de L-shuffling afin de sélectionner les meilleurs. Le test d'activité a été réduit à 2 heures (contre 17 heures pour le criblage des clones issus du premier tour d'évolution) pour tenir compte des améliorations obtenues avec le premier tour de L-shuffling.

L'activité des clones générés est comparée à l'activité obtenue avec le clone 154E4. Ce clone, issu du premier tour de L-shuffling, est celui qui a permis d'obtenir la meilleure amélioration d'activité.

Dans ces conditions de criblage, une amélioration de l'activité endoglucanase par rapport au clone de référence 154E4 (SEQ ID NO :10) a été trouvée dans plusieurs clones, dont notamment les clones 11G8 et 240H12.

### 2-Détermination de l'amélioration de l'activité endoglucanase

### 2-1/ Sur le substrat carboxy-méthyl cellulose (CMC)

Afin de déterminer le kcat, les activités des clones 11G8, 92A12 et 240H12 ont été mesurées par le test d'activité tel que décrit précédemment. La durée du test d'activité a été réduite à une heure d'incubation avec le substrat pour tenir compte des améliorations de ces clones.

Le TABLEAU 4 présente les valeurs du kcat ainsi que le facteur d'amélioration obtenus pour les clones 11G8, 92A12 et 240H12 par rapport au clone 154E4 (SEQ ID NO :10) dans ces conditions expérimentales.

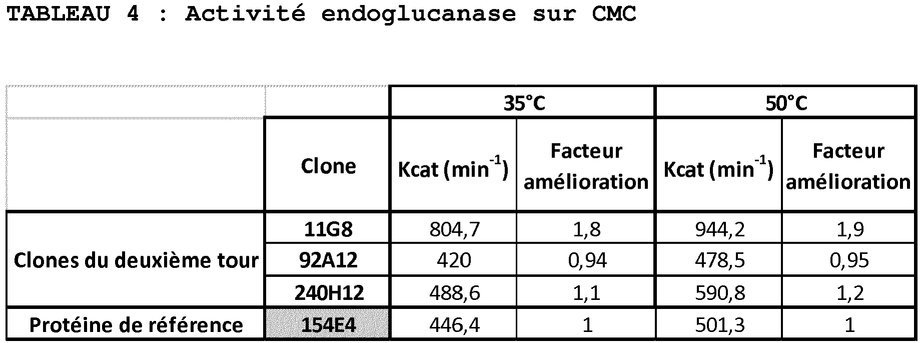

Les résultats montrent une amélioration d'activité par rapport au clone de référence 154E4 pour les clones 11G8 et 240H12.

### 2-2/ Sur le substrat phosphoric acid swollen cellulose (PASC)

L'amélioration d'activité des clones 11G8, 92A12 et 240H12 a ensuite été confirmée sur un second substrat : phosphoric acid swollen cellulose (PASC).

Afin de déterminer le kcat, l'activité de ces clones a été mesurée à 35 et 50°C par le test d'activité tel que décrit précédemment avec PASC comme substrat.

Le TABLEAU 5 présente les valeurs du kcat ainsi que le facteur d'amélioration obtenu pour les clones 11G8, 92A12 et 240H12 par rapport au clone 154E4 (SEQ ID NO :10) dans ces conditions expérimentales.

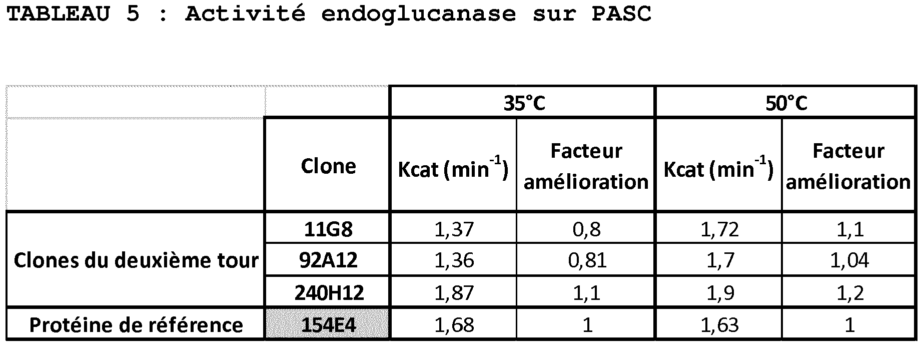

L'activité du clone 11G8 n'est pas améliorée à 35°C sur ce substrat par rapport au clone de référence 154E4. Par contre, l'activité du clone 11G8 est améliorée à 50°C. L'activité du clone 240H12 est améliorée à 35°C et à 50°C.

### EXEMPLE 3 : Clonage d'un variant endoglucanase 1 issu du premier tour de L-shuffling dans une souche T. reesei CL847 ΔEG1

La construction du fragment d'ADN à insérer dans *T*. *reesei*, contenant le clone 154E4 issu du premier tour du L-shuffling, a été réalisée par fusion PCR. Le fragment d'une longueur d'environ 5,4 kb était constitué du gène de la résistance à la phléomycine, de la séquence codante du clone 154E4 sous contrôle du promoteur et suivie par le terminateur *cbh1*. De la même manière, le gène de référence EG1 (SEQ ID NO :1) de *T. reesei* a été amplifié et fusionné entre le promoteur et terminateur *cbh1*, résultant en une deuxième construction.

Des protoplastes d'une souche *T. reesei* CL847 ΔEG1 ont été transformés selon une méthode classique connue de l'homme de l'art, par choc calcique et PEG, avec 5 µg de chaque construction, à savoir le fragment d'ADN contenant soit le gène 154E4 soit EG1. Les transformants ont été sélectionnés sur milieu sélectif PDA/saccharose contenant 30 pg/L de phléomycine. Quatorze clones de chaque transformation ont été repiqués. Après trois repiquages pour isoler des clones purs, sept clones ayant intégré le gène natif et cinq clones ayant intégré le variant 154E4 et sécrétant un niveau de protéines comparable à la souche CL847, ont finalement été obtenus.

### 1-Criblage à l'aide d'un test d'activité sur carboxyméthylcellulose (CMC)

Les 11 clones ont été mis en culture dans des plaques 24 puits contenant le milieu suivant :
800 µL H3PO4 85%, 4,2 g (NH4)2SO4, 0,3 g MgSO4.7H2O, 1,5 g CornSteep, 1 mL Oligo Ferment, 11,6 g acide maléique, 10 g SolkaFloc et 20 g lactose par litre de milieu. Le pH est ajusté à 5,8-6,0. Après 5 jours de culture à 30°C, le surnageant est prélevé et l'équivalent de 10 mg/L de protéines (mesurées par la méthode de Lowry) est utilisé pour un test d'activité sur CMC. 150 µL d'une solution CMC 2% dans du tampon citrate 50 mM pH 4,8 sont mélangés avec 150 µL de tampon citrate contenant 10 mg/L de protéines. La réaction est incubée à 50°C ou 35°C pendant 10 minutes, puis inactivée au bain-marie bouillant. Après centrifugation pendant 5 minutes, 20 µL de surnageant sont prélevés pour réaliser un dosage de sucres réducteurs à l'aide de l'acide 3,5-dinitrosalicylique (DNS). La réduction du DNS et la formation de l'acide 3-amino-5-nitrosalicylique sont suivis par lecture de l'absorption à 540 nm, et les sucres réducteurs quantifiés à l'aide d'une gamme de glucose.

Le TABLEAU 6 résume les activités obtenues avec les clones contenant le variant 154E4, en comparaison avec la souche de référence CL847, la souche CL847 ΔEG1, et la souche CL847 ΔEG1 retransformée avec le gène de référence EG1 (ΔEG1cEG1, moyenne obtenue avec les quatre meilleurs transformants).

**TABLEAU 6 : Activité endoglucanase sur CMC**

| **Clone** | **Activité spécifique 50°C (µmol/mg/min)** | **Rapport 154E4/ ΔEG1cEG1 (50°C)** | **Activité spécifique 35°C (µmol/mg/min)** | **Rapport 154E4/ ΔEG1cEG1 (35°C)** |
|---|---|---|---|---|
| **CL847** | 12,9 ± 3,1 | | 9,7 ± 0,4 | |
| **ΔEG1** | 6,4 ± 0,4 | | - | |
| **ΔEG1cEG1** | 16,8 ± 2,5 | | 12,1 ± 1,0 | |
| **154E4/2** | 22,5 ± 2,9 | 1,3 | 12,7 ± 2,0 | 1,1 |
| **154E4/8** | 24,1 ± 1,9 | 1,4 | 12,5 ± 2,0 | 1,0 |
| **154E4/9** | 20,2 ± 3,9 | 1,2 | 7,4 ± 2,2 | 0,6 |

Les résultats montrent que les activités des variants 154E4 sont entre 1,2 et 1,4 fois supérieures à celles des clones retransformés avec le gène de référence EG1 (SEQ ID NO :1). L'amélioration est visible à 35°C et à 50°C.

### 2-Clonage du variant 11G8 obtenu après le 2ème tour de L-shuffling dans une souche T. reesei CL847 ΔEG1 et criblage des transformants :

Le variant 11G8 a été cloné entre le promoteur et le terminateur cbh1 dans le plasmide pUT1040 contenant un gène de résistance à la phléomycine comme marqueur, à l'aide d'une double digestion BamH1/XhoI. 5 µg de ce vecteur ont été utilisés pour la transformation de la souche *T. reesei* CL847 ΔEG1. La transformation des protoplastes a été réalisée dans les mêmes conditions que pour le variant 154E4. A l'issue du processus de transformation et de trois repiquages successifs réalisés dans le but d'obtenir des clones purs, treize clones avec une production de protéines semblable à la souche CL847 ont été obtenus et ont été soumis au screening par mesure d'activité CMCase. Le test d'activité est identique au criblage des clones contenant le variant 154E4 du premier tour de L-shuffling. Six clones exprimant le variant 11G8 montrent une activité CMCase plus importante que celle de la souche ΔEG1cEG. Les deux meilleurs transformants ont une activité augmentée de 70% par rapport à la souche exprimant le gène de référence EG1 (SEQ ID NO :1).

Le TABLEAU 7 résume les activités obtenues avec les clones contenant le variant 11G8, en comparaison avec la souche de référence CL847, la souche CL847 ΔEG1, et la souche CL847 ΔEG1 retransformée avec le gène de référence EG1 (ΔEG1cEG1, moyenne obtenue avec les quatre meilleurs transformants).

**TABLEAU 7 : Activité endoglucanase sur CMC**

| **Clone** | **Activité spécifique 50°C (µmol/mg/min)** | **Rapport 11G8/ΔEG1 cEG1 (50°C)** | **Activité spécifique 35°C (µmol/mg/min)** | **Rapport 11G8/ ΔEG1cEG1 (35°C)** |
|---|---|---|---|---|
| **CL847** | 20,8 ± 0,8 | | 11,8 ± 0,5 | |
| **ΔEG1** | - | | 3,3 ± 1,1 | |
| **ΔEG1cEG1** | 13,2 ± 2,2 | | 9,1 ± 0,7 | |
| **11G8/6** | 15,6 ± 1,8 | 1,2 | 10,0 ± 0,4 | 1,1 |
| **11G8/7** | 17,3 ± 0,7 | 1,3 | 10,8 ± 0,9 | 1,2 |
| **11G8/9** | 17,9 ± 0,4 | 1,4 | 11,9 ± 0,2 | 1,3 |
| **11G8/10** | 15,0 ± 0,3 | 1,1 | 13,9 ± 1,6 | 1,5 |
| **11G8/12** | 17,0 ± 0,1 | 1,3 | 15,7 ± 0,8 | 1,7 |
| **11G8/13** | 17,3 ± 1,2 | 1,3 | 15,6 ± 0,4 | 1,7 |

Les résultats montrent que les activités des variants 11G8 sont entre 1,1 et 1,7 fois supérieures à celles des clones retransformés avec le gène de référence SEQ ID NO :1. L'amélioration est visible à 35°C et à 50°C.

### Exemple 4 : Expression recombinante de l'endoglucanase de référence EG1 et du variant amélioré 154E4 chez Saccharomyces cerevisiae

### 1- Production des protéines EG1 de référence et 154E4 dans le milieu extracellulaire

Les gènes de l'endoglucanase de *Trichoderma reesei* (EG1) et du variant 154E4 ont été clonés sans leur peptide signal dans le vecteur pESC-LeuαΔmyc (CNRS-CERMAV). Cette construction permet l'expression des protéines dans le milieu de culture de la souche *Saccharomyces cerevisiae* EBY100, auxotrophe pour la leucine et le tryptophane (Boder ET et Wittrup KD, Biotechnol Prog, 1998, 14:55-62). Ce plasmide permet de placer l'expression des gènes sous le contrôle du promoteur GAL1 inductible au galactose, et possède le gène marqueur de sélection de l'auxotrophie (Leu2) qui permet la sélection des transformants.

La transformation de *Saccharomyces cerevisiae* EBY100 a été réalisée selon les méthodes classiques connues de l'homme de l'art (transformation de levures par choc thermique et acétate de lithium). Les transformants ont été sélectionnés sur milieu YNB 0,67%-Glc 2%-Trp 0,01%.

Un transformant pour chaque gène (Scα-EG1 et Scα-154E4) a été utilisé pour ensemencer 15 mL d'un milieu minimum YNB 0,67%-Glc 2%-SD-Trp 0,01%. SD est un mélange d'acides aminés (40 mg/L d'adénine sulfate; 20 mg/L de L-arginine; 100 mg/L de L-acide aspartique; 100 mg/L de L-acide glutamique; 20 mg/L de L-histidine; 30 mg/L de L-lysine; 20 mg/L de L-méthionine; 50 mg/L de L-phénylalanine; 375 mg/L de L-sérine; 200 mg/L de L-thréonine; 30 mg/L de L-tyrosine; 150 mg/L de L-valine et 20 mg/L d'uracile). Après 24 heures de préculture à 30°C sous agitation à 220 rpm, les 2 souches Scα-EG1 et Scα-154E4 ont été utilisées pour ensemencer (DO₆₀₀ de 0,5) 150 mL de milieu YNB 0,67%-Gal 2%-SD-Trp 0,01%. Les cultures ont été incubées à 25°C sous agitation à 220 rpm. Après 8 heures d'incubation, 6 mL de citrate de sodium à pH 5,6 ont été ajoutés à chaque culture afin de stabiliser le pH à 5.

Après 4 jours d'incubation, 20 mL de culture ont été prélevés. Le surnageant de culture a été obtenu après centrifugation à 3000 g, à 4°C pendant 5 minutes.

### 2- Détermination de l'activité endoglucanase sur p-nitrophényl-β-lactoside

L'activité endoglucanase des surnageants de culture a été mesurée par hydrolyse du substrat *p*-nitrophenyl-β-lactoside (pNPL) dans un volume de 700 µL dans les conditions suivantes :
- 50 mM de tampon citrate à pH 5 ;
- 2 mM de pNPL ;
- 605 µL et 90 µL de surnageant de culture des souches Scα-154E4 ;
- Incubation à 35°C ou 50°C pendant 30 min pour la souche Scα-EG1.

La réaction a été arrêtée en ajoutant 100 µL de carbonate de sodium 1M à 100 µL du milieu réactionnel. La concentration en para-nitrophénol (pNP) libéré par hydrolyse du pNPL a été déterminée par mesure de l'absorbance à 415 nm par comparaison avec une gamme étalon de para-nitrophénol (linéaire de 0,36 µM à 360 µM).

Le TABLEAU 8 présente les résultats d'activité endoglucanase (AE en nmol.min⁻¹.mL⁻¹ de culture) sur pNPL à 35°C et 50°C des milieux de culture des souches ScαEG1 et SCα154E4.

**TABLEAU 8 : Activité endoglucanase sur pNPL à 35°C et 50°C des milieux de culture des souches ScαEG1 et SCα154E4.**

| | **AE à 35°C** | **AE à 50°C** | **Ratio d'activité 35°C/50°C** | **Amélioration de l'AE à 35°C** |
|---|---|---|---|---|
| **Scα-EG1** | 1,17 | 1,18 | 1 | - |
| **Scα-154E4** | 45,0 | 61,1 | 1,4 | 38,5 |

Les résultats obtenus montrent une amélioration à 35°C de l'activité enzymatique de la souche Scα-154E4 d'un facteur proche de 40 par rapport à la souche exprimant la protéine de référence EG1 de *T. reesei* (SEQ ID NO :2) . L'ampleur de l'amélioration d'activité constatée par rapport à *E. coli* et *T. reesei* suggère que l'enzyme a non seulement une activité spécifique améliorée mais qu'elle est également surexprimée et/ou mieux sécrétée.

### 3- Détermination de l'activité endoglucanase sur carboxyméthylcellulose

L'activité endoglucanase des surnageants de culture a été mesurée par hydrolyse de la carboxyméthylcellulose (CMC) dans un volume de 700 µL dans les conditions suivantes:
- 50 mM de tampon citrate à pH 5 ;
- 1% de CMC ;
- 210 µL de surnageant de culture des souches Scα-EG1 et Scα-154E4 respectivement dialysés contre du tampon citrate 50 mM pH 5 sur membrane de 10 kDa et concentrés 2 fois ;
- Incubation à 35°C pendant 48 heures.

La réaction a été arrêtée en ajoutant 150 µL de réactif au DNS à 100 µL du milieu réactionnel. Après chauffage pendant 5 minutes à 100°C et refroidissement dans la glace, la quantité de sucres réducteurs libérés a été déterminée par mesure de l'absorbance à 550 nm par comparaison avec une gamme étalon réalisée avec du glucose.

La FIGURE 1 présente les résultats d' hydrolyse de la CMC 1% par l'endoglucanase de référence EG1 (SEQ ID NO :2) et son mutant 154E4 (SEQ ID NO :10) respectivement sécrétées dans le milieu de culture des souches Scα-EG1 et Scα-154E4 .

Les résultats de la FIGURE 1 montrent que pendant les premières heures de réaction, la quantité de sucres réducteurs libérés par 1 mL de culture de la souche Scα-154E4 est environ 10 fois plus importante qu'avec 1 mL de Scα-EG1. L'ampleur de l'amélioration d'activité constatée par rapport à *E. coli* et *T. reesei* suggère que l'enzyme a non seulement une activité spécifique améliorée mais qu'elle est également surexprimée et/ou mieux sécrétée.

### Exemple 5 : Production d'enzymes par T. reesei en fioles alimentées

Les souches de référence et celles présentant la meilleure activité sur CMC (CL847, ΔEG1, ΔEG1cEG1, 154E4/2, 154E4/8, 11G8/10, 11G8/12, 11G8/13) ont été cultivées en fioles Erlenmeyer de 250 mL. 55 mL de milieu F45 (10 g/L de tampon dipotassium phtalate pH 6, 4.2g/L (NH₄)₂SO₄, 300 mg/L MgSO₄.7H₂O, 150 mg/l CaCl₂.2H₂O, 1,5 g/L cornsteep, 0,07% acide orthophosphorique, 5 mg/L FeSO₄, 1,4 mg/L MnSO₄, 1,4 mg/L ZnSO₄,3,7 mg/L CoCl₂ et 12,5 g/L glucose) sont ensemencés et agités à 150 rpm et 30°C. La production d'enzymes se fait en deux phases : une phase batch sur glucose et une phase fed-batch sur lactose. Des prélèvements réguliers permettent de déterminer le moment où la concentration de glucose passe en dessous de 3 g/L. A ce stade, une alimentation fed-batch à l'aide d'un pousse-seringues (6 voies) est lancée. Les cultures sont alimentées par une solution de 50 g/L lactose et 0,3% NH₃ à un débit de 40 mg de sucre / g de biomasse par heure. Des prélèvements journaliers sont réalisés pour déterminer le pH, le poids sec et la concentration de protéines dans le surnageant. Après 5 jours de culture en fed-batch, la culture est filtrée sur un filtre de 0,45 µm et le surnageant congelé.

La concentration finale de protéines a été de l'ordre de 3 à 4 g/L. Si la concentration était inférieure à 3 g/L, les surnageants ont été concentrés sur colonne (Vivaspin MWCO5, Sartorius).

### Exemple 6 : Efficacité des enzymes issus du L-shuffling en hydrolyse de biomasse lignocellulosique selon un procédé SHF

Le substrat de référence utilisé est une paille de blé ayant subi un prétraitement par explosion à la vapeur (19 bars - 3 minutes) après imprégnation acide à 0.01% en H₂SO₄ pendant 10 heures, lavée, neutralisée à pH 5, pressée, séchée. Le tableau 9 présente la composition du substrat de référence.

**Tableau 9 : Composition de la paille utilisée pour les essais d'hydrolyse**

| **Composition** | **% m / m** |
|---|---|
| WIS | 97, 52 |
| Teneur en cendres | 5 |
| Cellulose | 51,7 |
| Xylanes corrigés | 3,57 |
| Hémicellulose | 4,14 |
| Lignine de Klason (surestimée) | 36,49 |
| Acétyl | 0, 6 |

Les hydrolyses ont été réalisées à 10% de matière sèche m/m soit un équivalent de 5,4% de cellulose m/m. Le taux de protéines est fixé à 10 mg/g de matière sèche soit environ 19 mg/g de cellulose. La concentration des cocktails enzymatiques a été mesurée par la méthode de Lowry en utilisant la BSA comme référence. Chaque cocktail a été supplémenté en activité β-glucosidase à hauteur de 120 ± 2 UI/g de cellulose, en ajoutant de la β-glucosidase SP188 (Novozymes).

Les essais sont effectués en tubes Eppendorf de 2mL utile (1g réactionnel) contenant :
- 0,11 ± 0,001 g de substrat paille lavée
- 0,9 ± 0,02 mL de milieu réactionnel d'hydrolyse composé de tampon acétate 50 mM - pH 4,8 et chloramphénicol (0,05 g/L)
- entre 0,1 et 0,2 ± 0,02 g de cocktail enzymatique en fonction de leur taux de protéines.

Les hydrolyses enzymatiques sont réalisées à 45 ± 2 °C sous agitation type vortex à 900 rotations par minute dans un *Thermomixer Comfort Eppendorf.*

Tous les essais sont effectués en duplicat avec des temps de prélèvement fixés à t 24, 48 et 96 heures avec pour certains des prélèvements à t 72 heures.

A chaque temps de prélèvement, les hydrolysats sont ébouillantés 5 minutes dans les tubes Eppendorf sacrifiés. Ces tubes sont ensuite refroidis et centrifugés. Le dosage du glucose est effectué par HPLC. Parallèlement les résidus solides de chaque tube Eppendorf sont lavés et centrifugés 3 fois avant d'être séchés à 105°C pendant 24 heures de façon à évaluer les WIS (Water Insoluble Solids). Le calcul du rendement d'hydrolyse est effectué en tenant compte des WIS.

Les cocktails issus de l'exemple 5 ont été évalués. Deux tests témoins sont effectués avec les cocktails de référence supplémentés également en β-glucosidase pour comparaison : un cocktail produit par la souche CL847 ΔEG1 (ΔEG1) et un cocktail produit par la souche CL847 ΔEG1 retransformée avec le gène de référence EG1 (ΔEG1cEG1).

La FIGURE 2 présente les résultats de SHF pour le cocktail issu de la souche 154/8 exprimant le variant 154E4 (SEQ ID NO :10).

Les résultats présentés en FIGURE 2 montrent que la vitesse initiale d'hydrolyse du cocktail produit par le variant 154E4 est supérieure à celles des cocktails de référence ΔEG1 et ΔEG1cEG1. Le rendement final d'hydrolyse est également supérieur à celui des cocktails de référence ΔEG1 et ΔEG1cEG1.

La FIGURE 3 présente les résultats de SHF pour le cocktail issu de la souche 11G8/10 exprimant le variant 11G8 (SEQ ID NO :22).

Les résultats présentés en FIGURE 3 montrent que la vitesse initiale d'hydrolyse du cocktail produit par le variant 11G8 est supérieure à celles des cocktails de référence ΔEG1 et ΔEG1cEG1. Le rendement final d'hydrolyse est également supérieur à celui des cocktails de référence ΔEG1 et ΔEG1cEG1.

### Exemple 7 : Efficacité des enzymes en hydrolyse de biomasse lignocellulosique selon un procédé SSF

Le substrat utilisé est le même que celui décrit en tableau 9 (Exemple 6).

Les SSF sont réalisées en triplicat dans des réacteurs de laboratoire. Ils sont constitués des éléments suivants :
- un flacon en verre de 30 mL de volume utile ;
- un bouchon de sécurité en polyétheréthercétone (Peek) ;
- une soupape unidirectionnelle DV-118 commercialisée par la société Vaplock fixée à travers le bouchon. La soupape est configurée pour s'ouvrir en sortie lorsque la pression relative dans le flacon est supérieure à 70 mbar ;
- un tube creux en polypropylène, emmanché à travers un second traversant le bouchon et équipé au niveau de l'extrémité inférieure dudit tube d'un septum ;
- un joint plat disposé entre le goulot du flacon et le bouchon.

Le principe de mise en oeuvre des bioréacteurs est le suivant : le CO₂ produit lors de la fermentation éthanolique s'accumule dans le ciel situé au-dessus du milieu réactionnel entrainant par accumulation une augmentation de la pression dans le bioréacteur (P_{G}). Lorsque P_{G} devient supérieure à la pression d'ouverture de la soupape unidirectionnelle (P_{S}), celle-ci s'ouvre pour laisser échapper une quantité de gaz qui est par exemple déterminée par pesée. Lorsque P_{G} < P_{S}, la soupape se referme jusqu'à ce que P_{G} soit supérieur à P_{S}. Ainsi le bioréacteur en fonctionnement est toujours sous pression de manière à assurer un milieu anaérobie stable pour la fermentation. La quantité d'éthanol produite est évaluée par la production de CO₂ estimée par perte de poids à partir de l'équation stoechiométrique de fermentation du glucose en éthanol suivante :

C₆H₁₂O₆ (glucose) → 2 CO₂ + 2 CH₃CH₂OH (éthanol) + énergie

Le milieu de culture utilisé pour la SSF est un milieu aqueux qui comprend :
- un tampon acétate 50 mM pour pH 5 ;
- du chloramphénicol à 0,1 g/L ;
- du milieu nutritif à 3 g/L de KH₂PO₄, 2 g/L de (NH₄)₂SO₄, 0,4 g/L de MgSO₄,7H₂O et 1 g/L d'extrait de levure.

Les SSF ont été réalisées à 10 ± 0,01% m/m de matière sèche soit un équivalent de 5,4% cellulose m/m pour une masse réactionnelle totale de 15 ± 0,003 g. Le taux de protéines est fixé à 10 ± 0,01 mg de cellulases par gramme de matière sèche, soit environ 19 mg/g cellulose. La concentration des cocktails enzymatiques a été mesurée par la méthode de Lowry en utilisant la BSA (Bovine Serum Albumine) comme référence. Chaque cocktail a été supplémenté en activité β-glucosidase à hauteur de 120 ± 2 UI/g cellulose, en ajoutant de la β-glucosidase SP188 (Novozymes).

La levure de fermentation des sucres (*Saccharomyces cerevisiae*, souche Ethanol Red, Fermentis, France) est ajoutée dans le milieu pour obtenir une teneur de 2 ± 0,1 g/kg.

Les enzymes et les levures sont ajoutées dans les bioréacteurs après une heure de conditionnement de la paille de blé prétraitée à 35°C avec le tampon, le chloramphénicol et le milieu de culture.

La réaction de SSF est conduite à une température d'environ 35°C, en plaçant le bioréacteur de laboratoire dans un incubateur de type INFORS Multitron HT Standard avec une vitesse de rotation orbitale de 150 tours par minute.

Au cours du temps, la perte de masse a été suivie par pesée des bioréacteurs. A la fin de la réaction, le moût de fermentation est chauffé à 100°C pendant 5 minutes, refroidi et centrifugé pour séparer les matières solides non hydrolysées du jus de fermentation. Ce dernier est ensuite analysé par chromatographie en phase gazeuse afin de déterminer sa concentration en éthanol.

Les cocktails issus de l'exemple 5 ont été évalués. Deux tests témoins sont effectués avec les cocktails de référence supplémentés également en β-glucosidase pour comparaison : un cocktail produit par la souche CL847 ΔEG1 (ΔEG1) et un cocktail produit par la souche CL847 ΔEG1 retransformée avec le gène de référence EG1 (ΔEG1cEG1).

La FIGURE 4 présente les résultats de SSF pour les 2 cocktails exprimant l'endoglucanase 154E4 (moyenne des résultats obtenus avec les 2 variants).

Les résultats présentés en FIGURE 4 montrent que l'avancement (production d'éthanol pour la même dose d'enzymes) de la SSF sur 100 heures pour les 2 cocktails exprimant l'endoglucanase 154E4 est supérieur à ceux des cocktails de référence ΔEG1 et ΔEG1cEG1.

La FIGURE 5 présente les résultats de SSF pour les 3 cocktails exprimant l'endoglucanase 11G8 (moyenne moyenne des résultats obtenus avec les 2 variants).

Les résultats présentés en FIGURE 5 montrent que l'avancement de la SSF sur 100 heures pour les 3 cocktails exprimant l'endoglucanase 11G8 (moyenne) est supérieur à ceux des cocktails de référence ΔEG1 et ΔEG1cEG1.

## Revendications

1. Polypeptide isolé ou purifié **caractérisé en ce qu'**il a une activité endoglucanase améliorée par rapport à l'activité endoglucanase de la protéine de référence EG1, ledit polypeptide étant choisi dans le groupe consistant en :
i) une séquence d'acides aminés choisie parmi SEQ ID NO :6, SEQ ID NO :8, SEQ ID NO :10, SEQ ID NO :12, SEQ ID NO :14, SEQ ID NO :16, SEQ ID NO :18, SEQ ID NO :20, SEQ ID NO :22, SEQ ID NO :24 et SEQ ID NO :26;
ii) une séquence d'acides aminés présentant, par rapport à la séquence, SEQ ID NO :6, , SEQ ID NO :10, SEQ ID NO :18, SEQ ID NO :20, SEQ ID NO :22, SEQ ID NO :24 ou SEQ ID NO :26, un pourcentage d'identité d'au moins 90%, préférentiellement 95%, 98% ou 99%, ou une séquence d'acides aminés présentant, par rapport à la séquence SEQ ID NO :8, SEQ ID NO :12, SEQ ID NO :14 ou SEQ ID NO :16, un pourcentage d'identité d'au moins 98%.

2. Acide nucléique purifié ou isolé, **caractérisé en ce qu'**il code au moins un polypeptide selon la revendication **1.**

3. Acide nucléique purifié ou isolé selon la revendication 2 choisi parmi les séquences suivantes:, SEQ ID NO :5, SEQ ID NO :7, SEQ ID NO :9, SEQ ID NO :11; SEQ ID NO :13, SEQ ID NO :15, SEQ ID NO :17, SEQ ID NO :19, SEQ ID NO :21, SEQ ID NO :23 et SEQ ID NO :25.

4. Vecteur **caractérisé en ce qu'**il comprend un acide nucléique selon l'une des revendications **2 ou 3.**

5. Cellule hôte isolée **caractérisée en ce qu'**elle comprend l'acide nucléique selon l'une des revendications **2** ou **3** ou le vecteur selon la revendication **4.**

6. Cellule hôte isolée selon la revendication **5, caractérisée en ce qu'**elle est choisie parmi *Trichoderma, Aspergillus, Neurospora, Humicola, Pénicillium, Fusarium, Thermomonospora, Myceliophthora, Chrysosporium, Bacillus, Pseudomonas, Escherichia, Clostridium, Cellulomonas, Streptomyces, Yarrowia, Pichia* et *Saccharomyces.*

7. Cellule hôte isolée selon la revendication **5 ou 6, caractérisée en ce qu'**elle est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca, Saccharomyces cerevisiae.*

8. Utilisation dudit polypeptide selon la revendication **1** pour l'hydrolyse de la cellulose.

9. Utilisation dudit polypeptide selon la revendication **1** pour la production de biocarburant.

10. Composition enzymatique apte à agir sur la biomasse lignocellulosique, ladite composition enzymatique étant produite par des champignons filamenteux et comprenant au moins un polypeptide selon la revendication **1.**

11. Procédé de production de biocarburant à partir de la biomasse, **caractérisé en ce qu'**il comprend les étapes successives suivantes :
- mise en suspension en phase aqueuse de la biomasse à hydrolyser ;
- hydrolyse en présence d'une composition enzymatique selon la revendication **10** de la biomasse lignocellulosique de manière à produire un hydrolysat contenant du glucose ;
- fermentation du glucose de l'hydrolysat de manière à produire un moût de fermentation ;
- séparation du biocarburant du moût de fermentation.

12. Procédé de production de biocarburant à partir de la biomasse, **caractérisé en ce qu'**il comprend les étapes successives suivantes :
- mise en suspension en phase aqueuse de la biomasse à hydrolyser ;
- ajout simultané d'une composition enzymatique selon la revendication **10** et d'un organisme fermentaire de manière à produire un moût de fermentation ;
- séparation du biocarburant du moût de fermentation.

13. Procédé selon la revendication 12, dans lequel l'organisme fermentaire est choisi parmi une cellule hôte selon l'une des revendications 5 ou 6.

14. Procédé de production de biocarburant à partir de la biomasse, **caractérisé en ce qu'**il comprend les étapes successives suivantes :
- mise en suspension en phase aqueuse de la biomasse à hydrolyser ;
- ajout d'une ou plusieurs cellules hôtes selon l'une des revendications 5 à 7, avec un organisme fermentaire et/ou une composition enzymatique selon la revendication **10,** de manière à produire un moût de fermentation ;
- séparation du biocarburant du moût de fermentation.

## Patentansprüche

1. Isoliertes oder gereinigtes Polypeptid, **dadurch gekennzeichnet, dass** es eine verbesserte Endoglucanase-Aktivität aufweist im Vergleich zur Endoglucanase-Aktivität des Referenzproteins EG1, wobei das Polypeptid gewählt ist aus der Gruppe, umfassend:
i) eine Aminosäuresequenz, die gewählt ist aus SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 und SEQ ID NO: 26;
ii) eine Aminosäuresequenz, die im Vergleich zu der Sequenz SEQ ID NO: 6, SEQ ID NO: 10, SEQ ID NO: 18, SEQ ID NO: 20, SEQ ID NO: 22, SEQ ID NO: 24 oder SEQ ID NO: 26 eine Sequenzidentität von wenigstens 90%, bevorzugt 95%, 98% oder 99% aufweist, oder eine Aminosäuresequenz, die im Vergleich zu der Sequenz SEQ ID NO:8, SEQ ID NO: 12, SEQ ID NO: 14 oder SEQ ID NO: 16 eine Sequenzidentität von wenigstens 98% aufweist.

2. Gereinigte oder isolierte Nukleinsäure, **dadurch gekennzeichnet, dass** sie wenigstens ein Polypeptid nach Anspruch **1** kodiert.

3. Gereinigte oder isolierte Nukleinsäure nach Anspruch 2, welche gewählt ist aus den folgenden Sequenzen: SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO:23 und SEQ ID NO: 25.

4. Vektor, **dadurch gekennzeichnet, dass** er eine Nukleinsäure nach einem der Ansprüche **2 oder 3** umfasst.

5. Isolierte Wirtszelle, **dadurch gekennzeichnet, dass** sie die Nukleinsäure nach einem der Ansprüche **2** oder **3** oder den Vektor nach Anspruch **4** umfasst.

6. Isolierte Wirtszelle nach Anspruch **5, dadurch gekennzeichnet, dass** sie gewählt ist aus *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* und *Saccharomyces.*

7. Isolierte Wirtszelle nach Anspruch **5 oder 6,**
**dadurch gekennzeichnet, dass** sie gewählt ist aus *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca, Saccharomyces cerevisiae.*

8. Verwendung des Polypeptids nach Anspruch **1** zur Hydrolyse der Cellulose.

9. Verwendung des Polypeptids nach Anspruch **1** zur Herstellung von Biokraftstoff.

10. Enzymatische Zusammensetzung, welche in der Lage ist, auf lignocellulosehaltige Biomasse zu wirken, wobei die enzymatische Zusammensetzung durch filamentöse Pilze hergestellt wird und wenigstens ein Polypeptid nach Anspruch **1** umfasst.

11. Herstellungsverfahren für Biokraftstoff ausgehend von Biomasse, **dadurch gekennzeichnet, dass** es die folgenden aufeinanderfolgenden Schritte umfasst:
- Suspendieren der zu hydrolysierenden Biomasse in wässriger Phase;
- Hydrolyse der lignocellulosehaltigen Biomasse in Anwesenheit einer enzymatischen Zusammensetzung nach Anspruch **10** zur Herstellung eines glucosehaltigen Hydrolysats;
- Fermentation der Glucose des Hydrolysats zur Herstellung eines Fermentationsmosts ;
- Trennen des Biokraftstoffs vom Fermentationsmost.

12. Herstellungsverfahren für Biokraftstoff ausgehend von Biomasse, **dadurch gekennzeichnet, dass** es die folgenden aufeinanderfolgenden Schritte umfasst:
- Suspendieren der zu hydrolysierenden Biomasse in wässriger Phase;
- gleichzeitiges Hinzugeben einer enzymatischen Zusammensetzung nach Anspruch **10** und eines Fermentierungsorganismus zur Herstellung eines Fermentationsmostes;
- Trennen des Biokraftstoffs vom Fermentationsmost.

13. Verfahren nach Anspruch 12, bei welchem der Fermentierungsorganismus aus einer Wirtszelle nach einem der Ansprüche 5 oder 6 gewählt ist.

14. Herstellungsverfahren für Biokraftstoff ausgehend von Biomasse, **dadurch gekennzeichnet, dass** es die folgenden aufeinanderfolgenden Schritte umfasst:
- Suspendieren der zu hydrolysierenden Biomasse in wässriger Phase;
- Hinzufügen einer oder mehrerer Wirtszellen nach einem der Ansprüche 5 bis 7, mit einem Fermentierungsorganismus und/oder einer enzymatischen Zusammensetzung nach Anspruch **10** zur Herstellung eines Fermentationsmostes;
- Trennen des Biokraftstoffs vom Fermentationsmost.

## Claims

1. Isolated or purified polypeptide **characterised in that** it has an improved endoglucanase activity with respect to the endoglucanase activity of the reference protein EG1, said polypeptide being chosen from the group consisting of:
i) an amino acid sequence chosen from SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24 and SEQ ID NO: 26;
ii) an amino acid sequence having, with respect to the sequence, SEQ ID NO: 6, SEQ ID NO: 10, SEQ ID NO: 18, SEQ ID NO: 20, SEQ ID NO: 22, SEQ ID NO: 24 or SEQ ID NO: 26, a percentage identity of at least 90%, preferably 95%, 98% or 99%, or an amino acid sequence having, with respect to the sequence SEQ ID NO: 8, SEQ ID NO: 12, SEQ ID NO: 14 or SEQ ID NO: 16, a percentage identity of at least 98%.

2. Purified or isolated nucleic acid, **characterised in that** it encodes at least one polypeptide according to claim 1.

3. Purified or isolated nucleic acid according to claim 2 chosen from the following sequences: SEQ ID NO: 5, SEQ ID NO: 7, SEQ ID NO: 9, SEQ ID NO: 11; SEQ ID NO: 13, SEQ ID NO: 15, SEQ ID NO: 17, SEQ ID NO: 19, SEQ ID NO: 21, SEQ ID NO: 23 and SEQ ID NO: 25.

4. Vector **characterised in that** it comprises a nucleic acid according to one of claims 2 or 3.

5. Isolated host cell **characterised in that** it comprises nucleic acid according to one of claims 2 or 3 or the vector according to claim 4.

6. Isolated host cell according to claim 5, **characterised in that** it is chosen from *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia and Saccharomyces.*

7. Isolated host cell according to claim 5 or 6, **characterised in that** it is chosen from *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca, Saccharomyces cerevisiae.*

8. Use of said polypeptide according to claim 1 for the hydrolysis of cellulose.

9. Use of said polypeptide according to claim 1 for the production of biofuel.

10. Enzymatic composition able to act on the lignocellulosic biomass, said enzymatic composition being produced by filamentous fungi and comprising at least one polypeptide according to claim 1.

11. Method for producing biofuel from biomass, **characterised in that** it comprises the following successive steps:
- putting into suspension in aqueous phase the biomass to be hydrolysed;
- hydrolysis in the presence of an enzymatic composition according to claim 10 of the lignocellulosic biomass in such a way as to produce a hydrolysate containing glucose;
- fermentation of the glucose of the hydrolysate in such a way as to produce a fermentation must;
- separation of the biofuel from the fermentation must.

12. Method for producing biofuel from biomass, **characterised in that** it comprises the following successive steps:
- putting into suspension in aqueous phase the biomass to be hydrolysed;
- simultaneous adding of an enzymatic composition according to claim 10 and of a fermentation organism in such a way as to produce a fermentation must;
- separation of the biofuel from the fermentation must.

13. Method according to claim 12, wherein the fermentation organism is chosen from a host cell according to one of claims 5 or 6.

14. Method for producing biofuel from biomass, **characterised in that** it comprises the following successive steps:
- putting into suspension in aqueous phase the biomass to be hydrolysed;
- adding of one or several host cells according to one of claims 5 to 7, with a fermentation organism and/or enzymatic composition according to claim 10, in such a way as to produce a fermentation must;
- separation of the biofuel from the fermentation must.


### SEQUENCE LISTING

<110> IFPEN
<120> VARIANTS D'ENDOGLUCANASES A ACTIVITE AMELIOREE ET LEURS UTILISATIONS
<130> BFF
<160> 30
<170> PatentIn version 3.5
<210> 1
   <211> 1380
   <212> DNA
   <213> Trichoderma reesei
<400> 1
   atggcgccct cagttacact gccgttgacc acggccatcc tggccattgc ccggctcgtc      60
   gccgcccagc aaccgggtac cagcaccccc gaggtccatc ccaagttgac aacctacaag     120
   tgtacaaagt ccggggggtg cgtggcccag gacacctcgg tggtccttga ctggaactac     180
   cgctggatgc acgacgcaaa ctacaactcg tgcaccgtca acggcggcgt caacaccacg     240
   ctctgccctg acgaggcgac ctgtggcaag aactgcttca tcgagggcgt cgactacgcc     300
   gcctcgggcg tcacgacctc gggcagcagc ctcaccatga accagtacat gcccagcagc     360
   tctggcggct acagcagcgt ctctcctcgg ctgtatctcc tggactctga cggtgagtac     420
   gtgatgctga agctcaacgg ccaggagctg agcttcgacg tcgacctctc tgctctgccg     480
   tgtggagaga acggctcgct ctacctgtct cagatggacg agaacggggg cgccaaccag     540
   tataacacgg ccggtgccaa ctacgggagc ggctactgcg atgctcagtg ccccgtccag     600
   acatggagga acggcaccct caacactagc caccagggct tctgctgcaa cgagatggat     660
   atcctggagg gcaactcgag ggcgaatgcc ttgacccctc actcttgcac ggccacggcc     720
   tgcgactctg ccggttgcgg cttcaacccc tatggcagcg gctacaaaag ctactacggc     780
   cccggagata ccgttgacac ctccaagacc ttcaccatca tcacccagtt caacacggac     840
   aacggctcgc cctcgggcaa ccttgtgagc atcacccgca agtaccagca aaacggcgtc     900
   gacatcccca gcgcccagcc cggcggcgac accatctcgt cctgcccgtc cgcctcagcc     960
   tacggcggcc tcgccaccat gggcaaggcc ctgagcagcg gcatggtgct cgtgttcagc    1020
   atttggaacg acaacagcca gtacatgaac tggctcgaca gcggcaacgc cggcccctgc    1080
   agcagcaccg agggcaaccc atccaacatc ctggccaaca accccaacac gcacgtcgtc    1140
   ttctccaaca tccgctgggg agacattggg tctactacga actcgactgc gcccccgccc    1200
   ccgcctgcgt ccagcacgac gttttcgact acacggagga gctcgacgac ttcgagcagc    1260
   ccgagctgca cgcagactca ctgggggcag tgcggtggca ttgggtacag cgggtgcaag    1320
   acgtgcacgt cgggcactac gtgccagtat agcaacgact actactcgca atgcctttaa    1380
<210> 2
   <211> 459
   <212> PRT
   <213> Trichoderma reesei
<400> 2
   Met Ala Pro Ser Val Thr Leu Pro Leu Thr Thr Ala Ile Leu Ala Ile
   1               5                   10                  15
   Ala Arg Leu Val Ala Ala Gln Gln Pro Gly Thr Ser Thr Pro Glu Val
               20                  25                  30
   His Pro Lys Leu Thr Thr Tyr Lys Cys Thr Lys Ser Gly Gly Cys Val
           35                  40                  45
   Ala Gln Asp Thr Ser Val Val Leu Asp Trp Asn Tyr Arg Trp Met His
       50                  55                  60
   Asp Ala Asn Tyr Asn Ser Cys Thr Val Asn Gly Gly Val Asn Thr Thr
   65                  70                  75                  80
   Leu Cys Pro Asp Glu Ala Thr Cys Gly Lys Asn Cys Phe Ile Glu Gly
                   85                  90                  95
   Val Asp Tyr Ala Ala Ser Gly Val Thr Thr Ser Gly Ser Ser Leu Thr
               100                 105                 110
   Met Asn Gln Tyr Met Pro Ser Ser Ser Gly Gly Tyr Ser Ser Val Ser
           115                 120                 125
   Pro Arg Leu Tyr Leu Leu Asp Ser Asp Gly Glu Tyr Val Met Leu Lys
       130                 135                 140
   Leu Asn Gly Gln Glu Leu Ser Phe Asp Val Asp Leu Ser Ala Leu Pro
   145                 150                 155                 160
   Cys Gly Glu Asn Gly Ser Leu Tyr Leu Ser Gln Met Asp Glu Asn Gly
                   165                 170                 175
   Gly Ala Asn Gln Tyr Asn Thr Ala Gly Ala Asn Tyr Gly Ser Gly Tyr
               180                 185                 190
   Cys Asp Ala Gln Cys Pro Val Gln Thr Trp Arg Asn Gly Thr Leu Asn
           195                 200                 205
   Thr Ser His Gln Gly Phe Cys Cys Asn Glu Met Asp Ile Leu Glu Gly
       210                 215                 220
   Asn Ser Arg Ala Asn Ala Leu Thr Pro His Ser Cys Thr Ala Thr Ala
   225                 230                 235                 240
   Cys Asp Ser Ala Gly Cys Gly Phe Asn Pro Tyr Gly Ser Gly Tyr Lys
                   245                 250                 255
   Ser Tyr Tyr Gly Pro Gly Asp Thr Val Asp Thr Ser Lys Thr Phe Thr
               260                 265                 270
   Ile Ile Thr Gln Phe Asn Thr Asp Asn Gly Ser Pro Ser Gly Asn Leu
           275                 280                 285
   Val Ser Ile Thr Arg Lys Tyr Gln Gln Asn Gly Val Asp Ile Pro Ser
       290                 295                 300
   Ala Gln Pro Gly Gly Asp Thr Ile Ser Ser Cys Pro Ser Ala Ser Ala
   305                 310                 315                 320
   Tyr Gly Gly Leu Ala Thr Met Gly Lys Ala Leu Ser Ser Gly Met Val
                   325                 330                 335
   Leu Val Phe Ser Ile Trp Asn Asp Asn Ser Gln Tyr Met Asn Trp Leu
               340                 345                 350
   Asp Ser Gly Asn Ala Gly Pro Cys Ser Ser Thr Glu Gly Asn Pro Ser
           355                 360                 365
   Asn Ile Leu Ala Asn Asn Pro Asn Thr His Val Val Phe Ser Asn Ile
       370                 375                 380
   Arg Trp Gly Asp Ile Gly Ser Thr Thr Asn Ser Thr Ala Pro Pro Pro
   385                 390                 395                 400
   Pro Pro Ala Ser Ser Thr Thr Phe Ser Thr Thr Arg Arg Ser Ser Thr
                   405                 410                 415
   Thr Ser Ser Ser Pro Ser Cys Thr Gln Thr His Trp Gly Gln Cys Gly
               420                 425                 430
   Gly Ile Gly Tyr Ser Gly Cys Lys Thr Cys Thr Ser Gly Thr Thr Cys
           435                 440                 445
   Gln Tyr Ser Asn Asp Tyr Tyr Ser Gln Cys Leu
       450                 455
<210> 3
   <211> 1329
   <212> DNA
   <213> Artificial Sequence
<220>
   <223> 76B4
<400> 3
   cagcaaccgg gttcggtgac ccccgaggtc catcccaagt tgcccacctg gcagtgtaca       60
   aagtccgggg ggtgcgtgga gcaggacacc tcggtggtcc ttgactggaa ctaccgctgg      120
   ttccacacct cggacaacac cacctcgtgc accacgtcct cgggcatcga ctcgacgctc      180
   tgccctgacg cagcgacctg tgccgagaac tgcgtcgtgg agggcaccga ctacaccagc      240
   tcgggcatcg agacctcggg cagcagcctc accctgaggc agttcgtcaa ggacagcgag      300
   ggcaagacca acagcgtctc tcctcgggcc tatctcctgg gcgccgacgg tgactacgtg      360
   atgttcaagc tcctcaacca ggagctgagc ttcgacgtcg acgtctctac gctgccgtgt      420
   ggagagaacg cagcgctcta cttctctgag atggacaaga ccgggggccg gaacgagcac      480
   aacacgggcg gtgccaagta cgggagcggc tactgcgatg ctcagtgccc cgtccagaca      540
   tggaacaacg gcaccctcaa cactagccac cagggctcgt gctgcaacga gatggatatc      600
   ctggaggcca actcgagggc gaattcgtac acccctcacc cctgcagtgc cacggactgc      660
   gacaagggcg gttgcggctt caacccctat gctctcggcc aaaaaagcta ctggggcccc      720
   ggaggcaccg ttgacacctc caagcccttc accatcacca cccagttcat cacgaacgac      780
   ggcaccacca ccggcaccct ttccgaaatc cgccgccagt acatgcaaaa cggcaaggtg      840
   atcgccaatg ccgtttcctc cactggcgtc aactccatca ccgaggactg gtgcacgtcc      900
   gtcgacggct cggccgccac ctttggcggc ctcaccacca tgggcaaggc cctgggccgc      960
   ggcatggtgc tcatcttcag catttggaac gacgccagcg gctttatgaa ctggctcgac     1020
   agcggcaacg ccggcccctg cagcagcacc gagggcaacc cagacttgat caaggcccag     1080
   aaccccacga cgcacgtcgt cttctccaac atccgctggg gagacattgg gtctactttc     1140
   aagggttctg atggctcggt gacgacgacg acgtcgacta catcgaccaa gaccacgact     1200
   tcgaccgcgc cggggccaac gcagactcac tatgggcagt gcggtggcca agggtggact     1260
   gggcccacgg cttgcgcatc gccctacacg tgccaggttc tgaacccgtg gtactcgcaa     1320
   tgcctttaa                                                             1329
<210> 4
   <211> 442
   <212> PRT
   <213> Artificial Sequence
<220>
   <223> 76B4
<400> 4
   Gln Gln Pro Gly Ser Val Thr Pro Glu Val His Pro Lys Leu Pro Thr
   1               5                   10                  15
   Trp Gln Cys Thr Lys Ser Gly Gly Cys Val Glu Gln Asp Thr Ser Val
               20                  25                  30
   Val Leu Asp Trp Asn Tyr Arg Trp Phe His Thr Ser Asp Asn Thr Thr
           35                  40                  45
   Ser Cys Thr Thr Ser Ser Gly Ile Asp Ser Thr Leu Cys Pro Asp Ala
       50                  55                  60
   Ala Thr Cys Ala Glu Asn Cys Val Val Glu Gly Thr Asp Tyr Thr Ser
   65                  70                  75                  80
   Ser Gly Ile Glu Thr Ser Gly Ser Ser Leu Thr Leu Arg Gln Phe Val
                   85                  90                  95
   Lys Asp Ser Glu Gly Lys Thr Asn Ser Val Ser Pro Arg Ala Tyr Leu
               100                 105                 110
   Leu Gly Ala Asp Gly Asp Tyr Val Met Phe Lys Leu Leu Asn Gln Glu
           115                 120                 125
   Leu Ser Phe Asp Val Asp Val Ser Thr Leu Pro Cys Gly Glu Asn Ala
       130                 135                 140
   Ala Leu Tyr Phe Ser Glu Met Asp Lys Thr Gly Gly Arg Asn Glu His
   145                 150                 155                 160
   Asn Thr Gly Gly Ala Lys Tyr Gly Ser Gly Tyr Cys Asp Ala Gln Cys
                   165                 170                 175
   Pro Val Gln Thr Trp Asn Asn Gly Thr Leu Asn Thr Ser His Gln Gly
               180                 185                 190
   Ser Cys Cys Asn Glu Met Asp Ile Leu Glu Ala Asn Ser Arg Ala Asn
           195                 200                 205
   Ser Tyr Thr Pro His Pro Cys Ser Ala Thr Asp Cys Asp Lys Gly Gly
       210                 215                 220
   Cys Gly Phe Asn Pro Tyr Ala Leu Gly Gln Lys Ser Tyr Trp Gly Pro
   225                 230                 235                 240
   Gly Gly Thr Val Asp Thr Ser Lys Pro Phe Thr Ile Thr Thr Gln Phe
   245 250 255
   Ile Thr Asn Asp Gly Thr Thr Thr Gly Thr Leu Ser Glu Ile Arg Arg
   260 265 270
   Gln Tyr Met Gln Asn Gly Lys Val Ile Ala Asn Ala Val Ser Ser Thr
   275 280 285
   Gly Val Asn Ser Ile Thr Glu Asp Trp Cys Thr Ser Val Asp Gly Ser
   290 295 300
   Ala Ala Thr Phe Gly Gly Leu Thr Thr Met Gly Lys Ala Leu Gly Arg
   305 310 315 320
   Gly Met Val Leu Ile Phe Ser Ile Trp Asn Asp Ala Ser Gly Phe Met
   325 330 335
   Asn Trp Leu Asp Ser Gly Asn Ala Gly Pro Cys Ser Ser Thr Glu Gly
   340 345 350
   Asn Pro Asp Leu Ile Lys Ala Gln Asn Pro Thr Thr His Val Val Phe
   355 360 365
   Ser Asn Ile Arg Trp Gly Asp Ile Gly Ser Thr Phe Lys Gly Ser Asp
   370 375 380
   Gly Ser Val Thr Thr Thr Thr Ser Thr Thr Ser Thr Lys Thr Thr Thr
   385 390 395 400
   Ser Thr Ala Pro Gly Pro Thr Gln Thr His Tyr Gly Gln Cys Gly Gly
   405 410 415
   Gln Gly Trp Thr Gly Pro Thr Ala Cys Ala Ser Pro Tyr Thr Cys Gln
   420 425 430
   Val Leu Asn Pro Trp Tyr Ser Gln Cys Leu
   435 440
<210> 5
   <211> 1338
   <212> DNA
   <213> Artificial Sequence
<220>
   <223> 105F11
<400> 5
   cagcaaccgg gtaccagcac ccccgaggtc catcccaagt tgacaaccta caagtgtaca 60
   aagtccgggg ggtgcgtggc ccaggacacc tcggtggtcc ttgactggaa ctaccactgg 120
   attcacacgg tcgatgggta cacatcgtgc accacatcgt ccggcgtcga cagcacgctc 180
   tgccctgacg cggcgacctg tgcgaagaac tgcgtgatcg agccggccaa ctacaccagc 240
   gccggcgtca cgacctcggg cgacagcctc accatgtacc agtacgttca gagcaacggc 300
   gtctacacca acgcctctcc tcggctgtat ctcctgggcc ccgacaagaa ctacgtgatg 360
   ctgaagctcc taggccagga gctgaccttc gacgtcgacc tctctgctct gccgtgtgga 420
   gagaacggct cgctctacct gtctcagatg gacgagaacg ggggcgccaa ccagtataac 480
   acggccggtg ccaactacgg gagcggctac tgcgatgctc agtgccccgt ccagacatgg 540
   aggaacggca ccctcaacac tagccaccag ggctcgtgct gcaacgagat ggatatcctg 600
   gaggccaact cgaaggcgga agccttcacc cctcacccct gcatcggcga caactgcgac 660
   aagggcggtt gcggcttcaa cccctatgct ctcggccaaa aaagctactg gggccccgga 720
   ggcaccgttg acacctccaa gcccttcacc atcaccaccc agttcatcac gaacgacggc 780
   accaccaccg gcaccctttc cgaaatccgc cgccagtaca tgcaaaacgg caaggtgatc 840
   gccaatgccg tttcctccac tggcgtcaac tccatcaccg aggactggtg cacgtccgtc 900
   gacggctcgg ccgccacctt tggcggcctc accaccatgg gcaaggccct gggccgcggc 960
   atggtgctcg tgttcagcat ttggaacgac aacagccagt acatgaactg gctcgacagc 1020
   ggcaacgccg gcccctgcag cagcaccgag ggcaacccat ccaacatcct ggccaacaac 1080
   cccaacacgc acgtcgtctt ctccaacatc cgctggggag acattgggtc tactacgaac 1140
   tcgactgcgc ccccgccccc gcctgcgtcc agcacgacgt tttcgactac acggaggagc 1200
   tcgacgactt cgagcagccc gagctgcacg cagactcact gggggcagtg cggtggcatt 1260
   gggtacagcg ggtgcaagac gtgcacgtcg ggcactacgt gccagtatag caacgactac 1320
   tactcgcaat gcctttaa 1338
<210> 6
   <211> 445
   <212> PRT
   <213> Artificial Sequence
<220>
   <223> 105F11
<400> 6
   Gln Gln Pro Gly Thr Ser Thr Pro Glu Val His Pro Lys Leu Thr Thr
   1               5                   10                  15
   Tyr Lys Cys Thr Lys Ser Gly Gly Cys Val Ala Gln Asp Thr Ser Val
               20                  25                  30
   Val Leu Asp Trp Asn Tyr His Trp Ile His Thr Val Asp Gly Tyr Thr
           35                  40                  45
   Ser Cys Thr Thr Ser Ser Gly Val Asp Ser Thr Leu Cys Pro Asp Ala
       50                  55                  60
   Ala Thr Cys Ala Lys Asn Cys Val Ile Glu Pro Ala Asn Tyr Thr Ser
   65                  70                  75                  80
   Ala Gly Val Thr Thr Ser Gly Asp Ser Leu Thr Met Tyr Gln Tyr Val
                   85                  90                  95
   Gln Ser Asn Gly Val Tyr Thr Asn Ala Ser Pro Arg Leu Tyr Leu Leu
               100                 105                 110
   Gly Pro Asp Lys Asn Tyr Val Met Leu Lys Leu Leu Gly Gln Glu Leu
           115                 120                 125
   Thr Phe Asp Val Asp Leu Ser Ala Leu Pro Cys Gly Glu Asn Gly Ser
       130                 135                 140
   Leu Tyr Leu Ser Gln Met Asp Glu Asn Gly Gly Ala Asn Gln Tyr Asn
   145                 150                 155                 160
   Thr Ala Gly Ala Asn Tyr Gly Ser Gly Tyr Cys Asp Ala Gln Cys Pro
                   165                 170                 175
   Val Gln Thr Trp Arg Asn Gly Thr Leu Asn Thr Ser His Gln Gly Ser
               180                 185                 190
   Cys Cys Asn Glu Met Asp Ile Leu Glu Ala Asn Ser Lys Ala Glu Ala
           195                 200                 205
   Phe Thr Pro His Pro Cys Ile Gly Asp Asn Cys Asp Lys Gly Gly Cys
       210                 215                 220
   Gly Phe Asn Pro Tyr Ala Leu Gly Gln Lys Ser Tyr Trp Gly Pro Gly
   225                 230                 235                 240
   Gly Thr Val Asp Thr Ser Lys Pro Phe Thr Ile Thr Thr Gln Phe Ile
                   245                 250                 255
   Thr Asn Asp Gly Thr Thr Thr Gly Thr Leu Ser Glu Ile Arg Arg Gln
               260                 265                 270
   Tyr Met Gln Asn Gly Lys Val Ile Ala Asn Ala Val Ser Ser Thr Gly
           275                 280                 285
   Val Asn Ser Ile Thr Glu Asp Trp Cys Thr Ser Val Asp Gly Ser Ala
       290                 295                 300
   Ala Thr Phe Gly Gly Leu Thr Thr Met Gly Lys Ala Leu Gly Arg Gly
   305                 310                 315                 320
   Met Val Leu Val Phe Ser Ile Trp Asn Asp Asn Ser Gln Tyr Met Asn
                   325                 330                 335
   Trp Leu Asp Ser Gly Asn Ala Gly Pro Cys Ser Ser Thr Glu Gly Asn
               340                 345                 350
   Pro Ser Asn Ile Leu Ala Asn Asn Pro Asn Thr His Val Val Phe Ser
           355                 360                 365
   Asn Ile Arg Trp Gly Asp Ile Gly Ser Thr Thr Asn Ser Thr Ala Pro
       370                 375                 380
   Pro Pro Pro Pro Ala Ser Ser Thr Thr Phe Ser Thr Thr Arg Arg Ser
   385                 390                 395                 400
   Ser Thr Thr Ser Ser Ser Pro Ser Cys Thr Gln Thr His Trp Gly Gln
                   405                 410                 415
   Cys Gly Gly Ile Gly Tyr Ser Gly Cys Lys Thr Cys Thr Ser Gly Thr
               420                 425                 430
   Thr Cys Gln Tyr Ser Asn Asp Tyr Tyr Ser Gln Cys Leu
           435                 440                 445
<210> 7
   <211> 1131
   <212> DNA
   <213> Artificial Sequence
<220>
   <223> 107H12
<400> 7
   cagcaaccgg gttcggtgac ccccgaggtc catcccaagt tgacaaccta caagtgtaca      60
   aagtccgggg ggtgcgtggc ccaggacacc tcggtggtcc ttgactgggg ctaccactgg     120
   attcacacgg tcgatgggta cacatcgtgc accacatcgt ccggcgtcga cagcacgctc     180
   tgccctgacg cggcgacctg tgcgaagaac tgcgtgatcg agccggccaa ctacaccagc     240
   gccggcgtca cgacctcggg cgacagcctc accctgaggc agttcgtcaa ggacagcgag     300
   ggcaagacca acagcgtctc tcctcgggcc tatctcctgg gcgccgacgg tgactacgtg     360
   atgttcaagc tcctcaacca ggagctgagc ttcgacgtcg acctctctac gctgccgtgt     420
   ggagagaacg cagcgctcta cttctctgag atggacaaga ccgggggccg gaacgagcac     480
   aacacgggcg gtgccaagta cgggagcggc tactgcgatg ctcagtgccc cgtccagaca     540
   tggaacaacg gcaccctcaa cactagccac cagggctcgt gctgcaacga gatggatatc     600
   ctggaggcca actcgaaggc ggaagccttc acccctcacc cctgcatcgg cgacaactgc     660
   gacaagggcg gttgcggctt caactcgtat gcgcgcggca acaaaaacta ctgggcgccc     720
   ggaggcaccc tcgacacctc caagcccttc accatggtga cccagttcat cacggacgac     780
   ggcaccagct cgggcaagct tagccagatc gtgcgctcct acgtgcaaaa cggccagaag     840
   gtcgccagcg ccgtgtccgg cggcgacagc atcaccgtcg agggctgctc gtcctccgac     900
   gcctacggcg gcctcgtcgg tatgggcgag gccctgggcc gcggcatggt gctcgccatg     960
   agcatttgga acgacgccag cggcttcatg aactggctcg acagcggcga caacggcccc    1020
   tgcaacgaga ccgagggcga cccagccaac atcctggcca accaccccga ttcgcaggtc    1080
   gtcctgtcca acatccgctg gggagacatt gactctactg ttcagctcta a             1131
<210> 8
   <211> 376
   <212> PRT
   <213> Artificial Sequence
<220>
   <223> 107H12
<400> 8
   Gln Gln Pro Gly Ser Val Thr Pro Glu Val His Pro Lys Leu Thr Thr
   1               5                   10                  15
   Tyr Lys Cys Thr Lys Ser Gly Gly Cys Val Ala Gln Asp Thr Ser Val
               20                  25                  30
   Val Leu Asp Trp Gly Tyr His Trp Ile His Thr Val Asp Gly Tyr Thr
           35                  40                  45
   Ser Cys Thr Thr Ser Ser Gly Val Asp Ser Thr Leu Cys Pro Asp Ala
       50                  55                  60
   Ala Thr Cys Ala Lys Asn Cys Val Ile Glu Pro Ala Asn Tyr Thr Ser
   65                  70                  75                  80
   Ala Gly Val Thr Thr Ser Gly Asp Ser Leu Thr Leu Arg Gln Phe Val
                   85                  90                  95
   Lys Asp Ser Glu Gly Lys Thr Asn Ser Val Ser Pro Arg Ala Tyr Leu
               100                 105                 110
   Leu Gly Ala Asp Gly Asp Tyr Val Met Phe Lys Leu Leu Asn Gln Glu
           115                 120                 125
   Leu Ser Phe Asp Val Asp Leu Ser Thr Leu Pro Cys Gly Glu Asn Ala
       130                 135                 140
   Ala Leu Tyr Phe Ser Glu Met Asp Lys Thr Gly Gly Arg Asn Glu His
   145                 150                 155                 160
   Asn Thr Gly Gly Ala Lys Tyr Gly Ser Gly Tyr Cys Asp Ala Gln Cys
                   165                 170                 175
   Pro Val Gln Thr Trp Asn Asn Gly Thr Leu Asn Thr Ser His Gln Gly
               180                 185                 190
   Ser Cys Cys Asn Glu Met Asp Ile Leu Glu Ala Asn Ser Lys Ala Glu
           195                 200                 205
   Ala Phe Thr Pro His Pro Cys Ile Gly Asp Asn Cys Asp Lys Gly Gly
       210                 215                 220
   Cys Gly Phe Asn Ser Tyr Ala Arg Gly Asn Lys Asn Tyr Trp Ala Pro
   225                 230                 235                 240
   Gly Gly Thr Leu Asp Thr Ser Lys Pro Phe Thr Met Val Thr Gln Phe
                   245                 250                 255
   Ile Thr Asp Asp Gly Thr Ser Ser Gly Lys Leu Ser Gln Ile Val Arg
               260                 265                 270
   Ser Tyr Val Gln Asn Gly Gln Lys Val Ala Ser Ala Val Ser Gly Gly
           275                 280                 285
   Asp Ser Ile Thr Val Glu Gly Cys Ser Ser Ser Asp Ala Tyr Gly Gly
       290                 295                 300
   Leu Val Gly Met Gly Glu Ala Leu Gly Arg Gly Met Val Leu Ala Met
   305                 310                 315                 320
   Ser Ile Trp Asn Asp Ala Ser Gly Phe Met Asn Trp Leu Asp Ser Gly
                   325                 330                 335
   Asp Asn Gly Pro Cys Asn Glu Thr Glu Gly Asp Pro Ala Asn Ile Leu
               340                 345                 350
   Ala Asn His Pro Asp Ser Gln Val Val Leu Ser Asn Ile Arg Trp Gly
           355                 360                 365
   Asp Ile Asp Ser Thr Val Gln Leu
   370 375
<210> 9
   <211> 1320
   <212> DNA
   <213> Artificial Sequence
<220>
   <223> 154E4
<400> 9
   cagcaaccgg gtaccagcac ccccgaggtc catcccaagt tgacaaccta caagtgtaca 60
   actgctgggg ggtgcgtggc ccaggacacc tcggtggtcc ttgactgggg ctaccactgg 120
   attcacacgg tcgatgggta cacatcgtgc accacatcgt ccggcgtcga cagcacgctc 180
   tgccctgacg cggcgacctg tgcgaagaac tgcgtgatcg agccggccaa ctacaccagc 240
   gccggcgtca cgacctcggg cgacagcctc accatgtacc agtacgttca gagcaacggc 300
   gtctacacca acgcctctcc tcgggcctat ctcctgggcg ccgacggtga ctacgtgatg 360
   ttcaagctcc tcaaccagga gctgagcttc gacgtcgacg tctctacgct gccgtgtgga 420
   gagaacgcag cgctctactt ctctgagatg gacaagaccg ggggccggaa cgagcacaac 480
   acgggcggtg ccaagtacgg gagcggctac tgcgatgctc agtgccccgt ccagacatgg 540
   aacaacggca ccctcaacac tagccaccag ggctcgtgct gcaacgagat ggatatcctg 600
   gaggccaact cgaaggcgga agccttcacc cctcacccct gcatcggcga caactgcgac 660
   aagggcggtt gcggcttcaa ctcgtatgcg cgcggcaaca aaaactactg ggcgcccgga 720
   ggcaccctcg acacctccaa gcccttcacc atggtgaccc agttcatcac ggacgacggc 780
   accagctcgg gcaagcttag ccagatcgtg cgctcctacg tgcaaaacgg ccagaaggtc 840
   gccagcgccg tgtccggcgg cgacagcatc accgtcgagg gctgctcgtc ctccgacgcc 900
   tacggcggcc tcgtcggtat gggcgaggcc ctgggccgcg gcatggtgct cgtgttcagc 960
   atttggaacg acaacagcca gtacatgaac tggctcgaca gcggcaacgc cggcccctgc 1020
   agcagcaccg agggcaaccc atccaacatc ctggccaaca accccaacac gcacgtcgtc 1080
   ttctccaaca tccgctgggg agacattggg tctactacga actcgactgc gcccccgccc 1140
   ccgcctgcgt ccagcacgac gttttcgact acacggagga gctcgacgac ttcgagcagc 1200
   ccgagctgca cgcagactca ctgggggcag tgcggtggca ttgggtacag cgggtgcaag 1260
   acgtgcacgt cgggcactac gtgccagtat agcaacgact actactcgca atgcctttaa 1320
<210> 10
   <211> 439
   <212> PRT
   <213> Artificial Sequence
<220>
   <223> 154E4
<400> 10
   Gln Gln Pro Gly Thr Ser Thr Pro Glu Val His Pro Lys Leu Thr Thr
   1               5                   10                  15
   Tyr Lys Cys Thr Thr Ala Gly Gly Cys Val Ala Gln Asp Thr Ser Val
               20                  25                  30
   Val Leu Asp Trp Gly Tyr His Trp Ile His Thr Val Asp Gly Tyr Thr
           35                  40                  45
   Ser Cys Thr Thr Ser Ser Gly Val Asp Ser Thr Leu Cys Pro Asp Ala
       50                  55                  60
   Ala Thr Cys Ala Lys Asn Cys Val Ile Glu Pro Ala Asn Tyr Thr Ser
   65                  70                  75                  80
   Ala Gly Val Thr Thr Ser Gly Asp Ser Leu Thr Met Tyr Gln Tyr Val
                   85                  90                  95
   Gln Ser Asn Gly Val Tyr Thr Asn Ala Ser Pro Arg Ala Tyr Leu Leu
               100                 105                 110
   Gly Ala Asp Gly Asp Tyr Val Met Phe Lys Leu Leu Asn Gln Glu Leu
           115                 120                 125
   Ser Phe Asp Val Asp Val Ser Thr Leu Pro Cys Gly Glu Asn Ala Ala
       130                 135                 140
   Leu Tyr Phe Ser Glu Met Asp Lys Thr Gly Gly Arg Asn Glu His Asn
   145                 150                 155                 160
   Thr Gly Gly Ala Lys Tyr Gly Ser Gly Tyr Cys Asp Ala Gln Cys Pro
                   165                 170                 175
   Val Gln Thr Trp Asn Asn Gly Thr Leu Asn Thr Ser His Gln Gly Ser
               180                 185                 190
   Cys Cys Asn Glu Met Asp Ile Leu Glu Ala Asn Ser Lys Ala Glu Ala
           195                 200                 205
   Phe Thr Pro His Pro Cys Ile Gly Asp Asn Cys Asp Lys Gly Gly Cys
       210                 215                 220
   Gly Phe Asn Ser Tyr Ala Arg Gly Asn Lys Asn Tyr Trp Ala Pro Gly
   225                 230                 235                 240
   Gly Thr Leu Asp Thr Ser Lys Pro Phe Thr Met Val Thr Gln Phe Ile
                   245                 250                 255
   Thr Asp Asp Gly Thr Ser Ser Gly Lys Leu Ser Gln Ile Val Arg Ser
               260                 265                 270
   Tyr Val Gln Asn Gly Gln Lys Val Ala Ser Ala Val Ser Gly Gly Asp
           275                 280                 285
   Ser Ile Thr Val Glu Gly Cys Ser Ser Ser Asp Ala Tyr Gly Gly Leu
       290                 295                 300
   Val Gly Met Gly Glu Ala Leu Gly Arg Gly Met Val Leu Val Phe Ser
   305                 310                 315                 320
   Ile Trp Asn Asp Asn Ser Gln Tyr Met Asn Trp Leu Asp Ser Gly Asn
                   325                 330                 335
   Ala Gly Pro Cys Ser Ser Thr Glu Gly Asn Pro Ser Asn Ile Leu Ala
               340                 345                 350
   Asn Asn Pro Asn Thr His Val Val Phe Ser Asn Ile Arg Trp Gly Asp
           355                 360                 365
   Ile Gly Ser Thr Thr Asn Ser Thr Ala Pro Pro Pro Pro Pro Ala Ser
       370                 375                 380
   Ser Thr Thr Phe Ser Thr Thr Arg Arg Ser Ser Thr Thr Ser Ser Ser
   385                 390                 395                 400
   Pro Ser Cys Thr Gln Thr His Trp Gly Gln Cys Gly Gly Ile Gly Tyr
                   405                 410                 415
   Ser Gly Cys Lys Thr Cys Thr Ser Gly Thr Thr Cys Gln Tyr Ser Asn
               420                 425                 430
   Asp Tyr Tyr Ser Gln Cys Leu
           435
<210> 11
   <211> 1320
   <212> DNA
   <213> Artificial Sequence
<220>
   <223> 202C12
<400> 11
   cagcaaccgg gtaccagcac ccccgaggtc catcccaagt tgacaaccta caagtgtaca 60
   aagtccgggg ggtgcgtggc ccaggacacc tcggtggtcc ttgactggaa ctaccgctgg 120
   atgcacgacg caaactacaa ctcgtgcacc gtcaacggcg gcgtcaacac cacgctctgc 180
   cctgacgagg cgacctgtgg caagaactgc ttcatcgagg gcgtcgacta cgccgcctcg 240
   ggcgtcacga cctcgggcag cagcctcacc ctgaggcagt tcgtcaagga cagcgagggc 300
   aagaccaaca gcgtctctcc tcgggcctat ctcctgggcg ccgacggtga ctacgtgatg 360
   ttcaagctcc tcaaccagga gctgagcttc gacgtcgacg tctctacgct gccgtgtgga 420
   gagaacgcag cgctctactt ctctgagatg gacaagaccg ggggccggaa cgagcacaac 480
   acgggcggtg ccgagtacgg gagcggctac tgcgatgctc agtgccccgt ccagacatgg 540
   aacaacggca ccctcaacac tagccaccag ggctcgtgct gcaacgagat ggatatcctg 600
   gaggccaact cgaaggcgga agccttcacc cctcacccct gcatcggcga caactgcgac 660
   aagggcggtt gcggcttcaa ctcgtatgcg cgcggcaaca aaaactactg ggcgcccgga 720
   ggcaccctcg acacctccaa gcccttcacc atggtgaccc agttcatcac ggacgacggc 780
   accagctcgg gcaagcttag ccagatcgtg cgctcctacg tgcaaaacgg ccagaaggtc 840
   gccagcgccg tgtccggcgg cgacagcatc accgtcgagg gctgctcgtc ctccgacgcc 900
   tacggcggcc tcgtcggtat gggcgaggcc ctgggccgcg gcatggtgct cgccatgagc 960
   atttggaacg acgccagcgg cttcatgaac tggctcgaca gcggcgacaa cggcccctgc 1020
   aacgagaccg agggcaaccc atccaacatc ctggccaaca accccaacac gcacgtcgtc 1080
   ttctccaaca tccgctgggg agacattggg tctactacga actcgactgc gcccccgccc 1140
   ccgcctgcgt ccagcacgac gttttcgact acacggagga gctcgacgac ttcgagcagc 1200
   ccgagctgca cgcagactca ctgggggcag tgcggtggca ttgggtacag cgggtgcaag 1260
   acgtgcacgt cgggcactac gtgccagtat agcaacgact actactcgca atgcctttaa 1320
<210> 12
   <211> 439
   <212> PRT
   <213> Artificial Sequence
<220>
   <223> 202C12
<400> 12
   Gln Gln Pro Gly Thr Ser Thr Pro Glu Val His Pro Lys Leu Thr Thr
   1               5                   10                  15
   Tyr Lys Cys Thr Lys Ser Gly Gly Cys Val Ala Gln Asp Thr Ser Val
               20                  25                  30
   Val Leu Asp Trp Asn Tyr Arg Trp Met His Asp Ala Asn Tyr Asn Ser
           35                  40                  45
   Cys Thr Val Asn Gly Gly Val Asn Thr Thr Leu Cys Pro Asp Glu Ala
       50                  55                  60
   Thr Cys Gly Lys Asn Cys Phe Ile Glu Gly Val Asp Tyr Ala Ala Ser
   65                  70                  75                  80
   Gly Val Thr Thr Ser Gly Ser Ser Leu Thr Leu Arg Gln Phe Val Lys
                   85                  90                  95
   Asp Ser Glu Gly Lys Thr Asn Ser Val Ser Pro Arg Ala Tyr Leu Leu
               100                 105                 110
   Gly Ala Asp Gly Asp Tyr Val Met Phe Lys Leu Leu Asn Gln Glu Leu
           115                 120                 125
   Ser Phe Asp Val Asp Val Ser Thr Leu Pro Cys Gly Glu Asn Ala Ala
       130                 135                 140
   Leu Tyr Phe Ser Glu Met Asp Lys Thr Gly Gly Arg Asn Glu His Asn
   145                 150                 155                 160
   Thr Gly Gly Ala Glu Tyr Gly Ser Gly Tyr Cys Asp Ala Gln Cys Pro
                   165                 170                 175
   Val Gln Thr Trp Asn Asn Gly Thr Leu Asn Thr Ser His Gln Gly Ser
               180                 185                 190
   Cys Cys Asn Glu Met Asp Ile Leu Glu Ala Asn Ser Lys Ala Glu Ala
           195                 200                 205
   Phe Thr Pro His Pro Cys Ile Gly Asp Asn Cys Asp Lys Gly Gly Cys
       210                 215                 220
   Gly Phe Asn Ser Tyr Ala Arg Gly Asn Lys Asn Tyr Trp Ala Pro Gly
   225                 230                 235                 240
   Gly Thr Leu Asp Thr Ser Lys Pro Phe Thr Met Val Thr Gln Phe Ile
                   245                 250                 255
   Thr Asp Asp Gly Thr Ser Ser Gly Lys Leu Ser Gln Ile Val Arg Ser
               260                 265                 270
   Tyr Val Gln Asn Gly Gln Lys Val Ala Ser Ala Val Ser Gly Gly Asp
   275 280 285
   Ser Ile Thr Val Glu Gly Cys Ser Ser Ser Asp Ala Tyr Gly Gly Leu
   290 295 300
   Val Gly Met Gly Glu Ala Leu Gly Arg Gly Met Val Leu Ala Met Ser
   305 310 315 320
   Ile Trp Asn Asp Ala Ser Gly Phe Met Asn Trp Leu Asp Ser Gly Asp
   325 330 335
   Asn Gly Pro Cys Asn Glu Thr Glu Gly Asn Pro Ser Asn Ile Leu Ala
   340 345 350
   Asn Asn Pro Asn Thr His Val Val Phe Ser Asn Ile Arg Trp Gly Asp
   355 360 365
   Ile Gly Ser Thr Thr Asn Ser Thr Ala Pro Pro Pro Pro Pro Ala Ser
   370 375 380
   Ser Thr Thr Phe Ser Thr Thr Arg Arg Ser Ser Thr Thr Ser Ser Ser
   385 390 395 400
   Pro Ser Cys Thr Gln Thr His Trp Gly Gln Cys Gly Gly Ile Gly Tyr
   405 410 415
   Ser Gly Cys Lys Thr Cys Thr Ser Gly Thr Thr Cys Gln Tyr Ser Asn
   420 425 430
   Asp Tyr Tyr Ser Gln Cys Leu
   435
<210> 13
   <211> 1323
   <212> DNA
   <213> Artificial Sequence
<220>
   <223> 272A9
<400> 13
   cagcaaccgg gtaccagcac ccccgaggtc catcccaagt tgacaaccta caagtgtaca      60
   aagtccgggg ggtgcgtggc ccaggacacc tcggtggtcc ttgactggaa ctaccgctgg     120
   atgcacgacg caaactacaa ctcgtgcacc gtcaacggcg gcgtcaacac cacgctctgc     180
   cctgacgcgg cgacctgtgc gaagaactgc gtgatcgagc cggccaacta caccagcgcc     240
   ggcgtcacga cctcgggcga cagcctcacc atgtaccagt acgttcagag caacggcgtc     300
   tacaccaacg cctctcctcg gctgtatctc ctgggccccg acaagaacta cgtgatgctg     360
   aagctcctag gccaggagct gagcttcgac gtcgacctct ctgctctgcc gtgtggagag     420
   aacggcgccc tctacctgtc tgaaatgagc gccaccgggg gccgcaacga atataacacg     480
   ggcggtgccg agtacgggag cggctactgc gatgctcagt gccccgtcat cgcctggaag     540
   aacggcaccc tcaacactag cggcgcaagc tactgctgca acgagatgga tatcctggag     600
   gccaactcga gggcgaattc gtacacccct cacccctgca gtgccacgga ctgcgacaag     660
   ggcggttgcg gcttcaaccc ctatgctctc ggccaaaaaa gctactgggg ccccggaggc     720
   accgttgaca cctccaagcc cttcaccatc accacccagt tcatcacgaa cgacggcacc     780
   accaccggca ccctttccga aatccgccgc cagtacatgc aaaacggcaa ggtgatcgcc     840
   aatgccgttt cctccactgg cgtcaactcc atcaccgagg actggtgcac gtccgtcgac     900
   ggctcggccg ccacctttgg cggcctcacc accatgggca aggccctggg ccgcggcatg     960
   gtgctcatct tcagcatttg gaacgacgcc agcggcttta tgaactggct cgacagcggc    1020
   aacgccggcc cctgcagcag caccgagggc aacccagact tgatcaaggc ccagaacccc    1080
   acgacgcacg tcgtcttctc caacatccgc tggggagaca ttgggtctac tttcaagggt    1140
   tctgatggct cggtgacgac gacgacgtcg actacatcga ccaagaccac gacttcgacc    1200
   gcgccggggc caacgcagac tcactatggg cagtgcggtg gccaagggtg gactgggccc    1260
   acggcttgcg catcgcccta cacgtgccag gttctgaacc cgtggtactc gcaatgcctt    1320
   taa                                                                  1323
<210> 14
   <211> 440
   <212> PRT
   <213> Artificial Sequence
<220>
   <223> 272A9
<400> 14
   Gln Gln Pro Gly Thr Ser Thr Pro Glu Val His Pro Lys Leu Thr Thr
   1               5                   10                  15
   Tyr Lys Cys Thr Lys Ser Gly Gly Cys Val Ala Gln Asp Thr Ser Val
               20                  25                  30
   Val Leu Asp Trp Asn Tyr Arg Trp Met His Asp Ala Asn Tyr Asn Ser
           35                  40                  45
   Cys Thr Val Asn Gly Gly Val Asn Thr Thr Leu Cys Pro Asp Ala Ala
       50                  55                  60
   Thr Cys Ala Lys Asn Cys Val Ile Glu Pro Ala Asn Tyr Thr Ser Ala
   65 70 75 80
   Gly Val Thr Thr Ser Gly Asp Ser Leu Thr Met Tyr Gln Tyr Val Gln
   85 90 95
   Ser Asn Gly Val Tyr Thr Asn Ala Ser Pro Arg Leu Tyr Leu Leu Gly
   100 105 110
   Pro Asp Lys Asn Tyr Val Met Leu Lys Leu Leu Gly Gln Glu Leu Ser
   115 120 125
   Phe Asp Val Asp Leu Ser Ala Leu Pro Cys Gly Glu Asn Gly Ala Leu
   130 135 140
   Tyr Leu Ser Glu Met Ser Ala Thr Gly Gly Arg Asn Glu Tyr Asn Thr
   145 150 155 160
   Gly Gly Ala Glu Tyr Gly Ser Gly Tyr Cys Asp Ala Gln Cys Pro Val
   165 170 175
   Ile Ala Trp Lys Asn Gly Thr Leu Asn Thr Ser Gly Ala Ser Tyr Cys
   180 185 190
   Cys Asn Glu Met Asp Ile Leu Glu Ala Asn Ser Arg Ala Asn Ser Tyr
   195 200 205
   Thr Pro His Pro Cys Ser Ala Thr Asp Cys Asp Lys Gly Gly Cys Gly
   210 215 220
   Phe Asn Pro Tyr Ala Leu Gly Gln Lys Ser Tyr Trp Gly Pro Gly Gly
   225 230 235 240
   Thr Val Asp Thr Ser Lys Pro Phe Thr Ile Thr Thr Gln Phe Ile Thr
   245 250 255
   Asn Asp Gly Thr Thr Thr Gly Thr Leu Ser Glu Ile Arg Arg Gln Tyr
   260 265 270
   Met Gln Asn Gly Lys Val Ile Ala Asn Ala Val Ser Ser Thr Gly Val
   275 280 285
   Asn Ser Ile Thr Glu Asp Trp Cys Thr Ser Val Asp Gly Ser Ala Ala
   290 295 300
   Thr Phe Gly Gly Leu Thr Thr Met Gly Lys Ala Leu Gly Arg Gly Met
   305 310 315 320
   Val Leu Ile Phe Ser Ile Trp Asn Asp Ala Ser Gly Phe Met Asn Trp
                   325                 330                 335
   Leu Asp Ser Gly Asn Ala Gly Pro Cys Ser Ser Thr Glu Gly Asn Pro
               340                 345                 350
   Asp Leu Ile Lys Ala Gln Asn Pro Thr Thr His Val Val Phe Ser Asn
           355                 360                 365
   Ile Arg Trp Gly Asp Ile Gly Ser Thr Phe Lys Gly Ser Asp Gly Ser
       370                 375                 380
   Val Thr Thr Thr Thr Ser Thr Thr Ser Thr Lys Thr Thr Thr Ser Thr
   385                 390                 395                 400
   Ala Pro Gly Pro Thr Gln Thr His Tyr Gly Gln Cys Gly Gly Gln Gly
                   405                 410                 415
   Trp Thr Gly Pro Thr Ala Cys Ala Ser Pro Tyr Thr Cys Gln Val Leu
               420                 425                 430
   Asn Pro Trp Tyr Ser Gln Cys Leu
           435                 440
<210> 15
   <211> 1317
   <212> DNA
   <213> Artificial Sequence
<220>
   <223> 278F10
<400> 15
   cagcaaccgg gtaccagcac ccccgaggtc catcccaagt tgacaaccta caagtgtaca        60
   aagtccgggg ggtgcgtgga gcaggacacc tcggtggtcc ttgactggaa ctaccgctgg       120
   ttccacacct cggacaacac cacctcgtgc accacgtcct cgggcatcga ctcgacgctc       180
   tgccctgacg cagcgacctg tgccgagaac tgcgtcgtgg agggcaccga ctacaccagc       240
   tcgggcatcg agacctcggg cagcagcctc accctgaggc agttcgtcaa ggacagcgag       300
   ggcaagacca acagcgtctc tcctcgggcc tatctcctgg gcgccgacgg tgactacgtg       360
   atgttcaagc tcctcaacca ggagctgagc ttcgacgtcg acgtctctac gctgccgtgt       420
   ggagagaacg cagcgctcta cttctctgag atggacaaga ccgggggccg gaacgagcac       480
   aacacgggcg gtgccaagta cgggagcggc tactgcgatg ctcagtgccc cgtccagaca       540
   tggaacaacg gcaccctcaa cactagccac cagggctcgt gctgcaacga gatggatatc       600
   ctggaggcca actcgaaggc ggaagccttc acccctcacc cctgcatcgg cgacaactgc       660
   gacaagggcg gttgcggctt caactcgtat gcgcgcggca acaaaaacta ctgggcgccc       720
   ggaggcaccc tcgacacctc caagcccttc accatggtga cccagttcat cacggacgac       780
   ggcaccagct cgggcaacct tgtgagcatc acccgcaagt accagcaaaa cggcgtcgac       840
   atccccagcg cccagcccgg cggcgacacc atctcgtcct gcccgtccgc ctcagcctac       900
   ggcggcctcg ccaccatggg caaggccctg agcagcggca tggtgctcgt gttcagcatt       960
   tggaacgaca acagccagta catgaactgg ctcgacagcg gcaacgccgg cccctgcagc      1020
   agcaccgagg gcaacccatc caacatcctg gccaacaacc ccaacacgca cgtcgtcttc      1080
   tccaacatcc gctggggaga cattgggtct actacgaact cgactgcgcc cccgcccccg      1140
   cctgcgtcca gcacgacgtt ttcgactaca cggaggagct cgacgacttc gagcagcccg      1200
   agctgcacgc agactcactg ggggcagtgc ggtggcattg ggtacagcgg gtgcaagacg      1260
   tgcacgtcgg gcactacgtg ccagtatagc aacgactact actcgcaatg cctttaa         1317
<210> 16
   <211> 438
   <212> PRT
   <213> Artificial Sequence
<220>
   <223> 278F10
<400> 16
   Gln Gln Pro Gly Thr Ser Thr Pro Glu Val His Pro Lys Leu Thr Thr
   1               5                   10                  15
   Tyr Lys Cys Thr Lys Ser Gly Gly Cys Val Glu Gln Asp Thr Ser Val
               20                  25                  30
   Val Leu Asp Trp Asn Tyr Arg Trp Phe His Thr Ser Asp Asn Thr Thr
           35                  40                  45
   Ser Cys Thr Thr Ser Ser Gly Ile Asp Ser Thr Leu Cys Pro Asp Ala
       50                  55                  60
   Ala Thr Cys Ala Glu Asn Cys Val Val Glu Gly Thr Asp Tyr Thr Ser
   65                  70                  75                  80
   Ser Gly Ile Glu Thr Ser Gly Ser Ser Leu Thr Leu Arg Gln Phe Val
                   85                  90                  95
   Lys Asp Ser Glu Gly Lys Thr Asn Ser Val Ser Pro Arg Ala Tyr Leu
               100                 105                 110
   Leu Gly Ala Asp Gly Asp Tyr Val Met Phe Lys Leu Leu Asn Gln Glu
           115                 120                 125
   Leu Ser Phe Asp Val Asp Val Ser Thr Leu Pro Cys Gly Glu Asn Ala
       130                 135                 140
   Ala Leu Tyr Phe Ser Glu Met Asp Lys Thr Gly Gly Arg Asn Glu His
   145                 150                 155                 160
   Asn Thr Gly Gly Ala Lys Tyr Gly Ser Gly Tyr Cys Asp Ala Gln Cys
                   165                 170                 175
   Pro Val Gln Thr Trp Asn Asn Gly Thr Leu Asn Thr Ser His Gln Gly
               180                 185                 190
   Ser Cys Cys Asn Glu Met Asp Ile Leu Glu Ala Asn Ser Lys Ala Glu
           195                 200                 205
   Ala Phe Thr Pro His Pro Cys Ile Gly Asp Asn Cys Asp Lys Gly Gly
       210                 215                 220
   Cys Gly Phe Asn Ser Tyr Ala Arg Gly Asn Lys Asn Tyr Trp Ala Pro
   225                 230                 235                 240
   Gly Gly Thr Leu Asp Thr Ser Lys Pro Phe Thr Met Val Thr Gln Phe
                   245                 250                 255
   Ile Thr Asp Asp Gly Thr Ser Ser Gly Asn Leu Val Ser Ile Thr Arg
               260                 265                 270
   Lys Tyr Gln Gln Asn Gly Val Asp Ile Pro Ser Ala Gln Pro Gly Gly
           275                 280                 285
   Asp Thr Ile Ser Ser Cys Pro Ser Ala Ser Ala Tyr Gly Gly Leu Ala
       290                 295                 300
   Thr Met Gly Lys Ala Leu Ser Ser Gly Met Val Leu Val Phe Ser Ile
   305                 310                 315                 320
   Trp Asn Asp Asn Ser Gln Tyr Met Asn Trp Leu Asp Ser Gly Asn Ala
                   325                 330                 335
   Gly Pro Cys Ser Ser Thr Glu Gly Asn Pro Ser Asn Ile Leu Ala Asn
               340                 345                 350
   Asn Pro Asn Thr His Val Val Phe Ser Asn Ile Arg Trp Gly Asp Ile
           355                 360                 365
   Gly Ser Thr Thr Asn Ser Thr Ala Pro Pro Pro Pro Pro Ala Ser Ser
       370                 375                 380
   Thr Thr Phe Ser Thr Thr Arg Arg Ser Ser Thr Thr Ser Ser Ser Pro
   385                 390                 395                 400
   Ser Cys Thr Gln Thr His Trp Gly Gln Cys Gly Gly Ile Gly Tyr Ser
                   405                 410                 415
   Gly Cys Lys Thr Cys Thr Ser Gly Thr Thr Cys Gln Tyr Ser Asn Asp
               420                 425                 430
   Tyr Tyr Ser Gln Cys Leu
           435
<210> 17
   <211> 1323
   <212> DNA
   <213> Artificial Sequence
<220>
   <223> 293B2
<400> 17
   cagcaaccgg gttcggtgac ccccgaggtc catcccaagt tgcccacctg gcagtgtaca      60
   aagtccgggg ggtgcgtgga gcaggacacc tcggtggtcc ttgactggaa ctaccgctgg     120
   ttccacacct cggacaacac cacctcgtgc accacgtcct cgggcatcga ctcgacgctc     180
   tgccctgacg cagcgacctg tgccgagaac tgcgtcgtgg agggcaccga ctacaccagc     240
   tcgggcatcg agacctcggg cagcagcctc accctgaggc agttcgtcaa ggacagcgag     300
   ggcaagacca acagcgtctc tcctcgggcc tatctcctgg gcgccgacgg tgactacgtg     360
   atgttcaagc tcctcaacca ggagctgagc ttcgacgtcg acgtctctac gctgccgtgt     420
   ggagagaacg cagcgctcta cttctctgag atggacaaga ccgggggccg gaacgagcac     480
   aacacgggcg gtgccaagta cgggagcggc tactgcgatg ctcagtgccc cgtccagaca     540
   tggaacaacg gcaccctcaa cactagccac cagggctcgt gctgcaacga gatggatatc     600
   ctggaggcca actcgaaggc ggaagccttc acccctcacc cctgcatcgg cgacaactgc     660
   gacaagggcg gttgcggctt caactcgtat gcgcgcggca acaaaaacta ctgggcgccc     720
   ggaggcaccc tcgacacctc caagcccttc accatggtga cccagttcat cacggacgac     780
   ggcaccagct cgggcaagct tagccagatc gtgcgctcct acgtgcaaaa cggccagaag     840
   gtcgccagcg ccgtgtccgg cggcgacagc atcaccgtcg agggctgctc gtcctccgac     900
   gcctacggcg gcctcgtcgg tatgggcgag gccctgggcc gcggcatggt gctcgtgttc     960
   agcatttgga acgacaacag ccagtacatg aactggctcg acagcggcaa cgccggcccc    1020
   tgcagcagca ccgagggcaa cccatccaac atcctggcca acaaccccaa cacgcacgtc    1080
   gtcttctcca acatccgctg gggagacatt gggtctacta cgaactcgac tgcgcccccg    1140
   cccccgcctg cgtccagcac gacgttttcg actacacgga ggagctcgac gacttcgagc    1200
   agcccgagct gcacgcagac tcactggggg cagtgcggtg gcattgggta cagcgggtgc    1260
   aagacgtgca cgtcgggcac tacgtgccag tatagcaacg actactactc gcaatgcctt    1320
   taa                                                                 1323
<210> 18
   <211> 440
   <212> PRT
   <213> Artificial Sequence
<220>
   <223> 293B2
<400> 18
   Gln Gln Pro Gly Ser Val Thr Pro Glu Val His Pro Lys Leu Pro Thr
   1               5                   10                  15
   Trp Gln Cys Thr Lys Ser Gly Gly Cys Val Glu Gln Asp Thr Ser Val
               20                  25                  30
   Val Leu Asp Trp Asn Tyr Arg Trp Phe His Thr Ser Asp Asn Thr Thr
           35                  40                  45
   Ser Cys Thr Thr Ser Ser Gly Ile Asp Ser Thr Leu Cys Pro Asp Ala
       50                  55                  60
   Ala Thr Cys Ala Glu Asn Cys Val Val Glu Gly Thr Asp Tyr Thr Ser
   65                  70                  75                  80
   Ser Gly Ile Glu Thr Ser Gly Ser Ser Leu Thr Leu Arg Gln Phe Val
                   85                  90                  95
   Lys Asp Ser Glu Gly Lys Thr Asn Ser Val Ser Pro Arg Ala Tyr Leu
               100                 105                 110
   Leu Gly Ala Asp Gly Asp Tyr Val Met Phe Lys Leu Leu Asn Gln Glu
           115                 120                 125
   Leu Ser Phe Asp Val Asp Val Ser Thr Leu Pro Cys Gly Glu Asn Ala
       130                 135                 140
   Ala Leu Tyr Phe Ser Glu Met Asp Lys Thr Gly Gly Arg Asn Glu His
   145                 150                 155                 160
   Asn Thr Gly Gly Ala Lys Tyr Gly Ser Gly Tyr Cys Asp Ala Gln Cys
   165 170 175
   Pro Val Gln Thr Trp Asn Asn Gly Thr Leu Asn Thr Ser His Gln Gly
   180 185 190
   Ser Cys Cys Asn Glu Met Asp Ile Leu Glu Ala Asn Ser Lys Ala Glu
   195 200 205
   Ala Phe Thr Pro His Pro Cys Ile Gly Asp Asn Cys Asp Lys Gly Gly
   210 215 220
   Cys Gly Phe Asn Ser Tyr Ala Arg Gly Asn Lys Asn Tyr Trp Ala Pro
   225 230 235 240
   Gly Gly Thr Leu Asp Thr Ser Lys Pro Phe Thr Met Val Thr Gln Phe
   245 250 255
   Ile Thr Asp Asp Gly Thr Ser Ser Gly Lys Leu Ser Gln Ile Val Arg
   260 265 270
   Ser Tyr Val Gln Asn Gly Gln Lys Val Ala Ser Ala Val Ser Gly Gly
   275 280 285
   Asp Ser Ile Thr Val Glu Gly Cys Ser Ser Ser Asp Ala Tyr Gly Gly
   290 295 300
   Leu Val Gly Met Gly Glu Ala Leu Gly Arg Gly Met Val Leu Val Phe
   305 310 315 320
   Ser Ile Trp Asn Asp Asn Ser Gln Tyr Met Asn Trp Leu Asp Ser Gly
   325 330 335
   Asn Ala Gly Pro Cys Ser Ser Thr Glu Gly Asn Pro Ser Asn Ile Leu
   340 345 350
   Ala Asn Asn Pro Asn Thr His Val Val Phe Ser Asn Ile Arg Trp Gly
   355 360 365
   Asp Ile Gly Ser Thr Thr Asn Ser Thr Ala Pro Pro Pro Pro Pro Ala
   370 375 380
   Ser Ser Thr Thr Phe Ser Thr Thr Arg Arg Ser Ser Thr Thr Ser Ser
   385 390 395 400
   Ser Pro Ser Cys Thr Gln Thr His Trp Gly Gln Cys Gly Gly Ile Gly
   405 410 415
   Tyr Ser Gly Cys Lys Thr Cys Thr Ser Gly Thr Thr Cys Gln Tyr Ser
               420                 425                 430
   Asn Asp Tyr Tyr Ser Gln Cys Leu
           435                 440
<210> 19
   <211> 1323
   <212> DNA
   <213> Artificial Sequence
<220>
   <223> 309A11
<400> 19
   cagcaaccgg gttcggtgac ccccgaggtc catcccaagt tgcccacctg gcagtgtaca      60
   aagtccgggg ggtgcgtgga gcaggacacc tcggtggtcc ttgactggaa ctaccgctgg     120
   ttccacacct cggacaacac cacctcgtgc accacgtcct cgggcatcga ctcgacgctc     180
   tgccctgacg cagcgacctg tgccgagaac tgcgtcgtgg agggcaccga ctacaccagc     240
   tcgggcatcg agacctcggg cagcagcctc accctgaggc agttcgtcaa ggacagcgag     300
   ggcaagacca acagcgtctc tcctcgggcc tatctcctgg gcgccgacgg tgactacgtg     360
   atgttcaagc tcctcaacca ggagctgagc ttcgacgtcg acgtctctac gctgccgtgt     420
   ggagagaacg cagcgctcta cttctctgag atggacaaga ccgggggccg gaacgagcac     480
   aacacgggcg gtgccaagta cgggagcggc tactgcgatg ctcagtgccc cgtccagaca     540
   tggaacaacg gcaccctcaa cactagccac cagggctcgt gctgcaacga gatggatatc     600
   ctggaggcca actcgaaggc ggaagccttc acccctcacc cctgcatcgg cgacaactgc     660
   gacaagggcg gttgcggctt caactcgtat gcgcgcggca acaaaaacta ctgggcgccc     720
   ggaggcaccc tcgacacctc caagcccttc accatggtga cccagttcat cacggacgac     780
   ggcaccagct cgggcaagct tagccagatc gtgcgctcct acgtgcaaaa cggccagaag     840
   gtcgccagcg ccgtgtccgg cggcgacagc atcaccgtcg agggctgctc gtccgcctca     900
   gcctacggcg gcctcgccac catgggcaag gccctgagca gcggcatggt gctcgtgttc     960
   agcatttgga acgacaacag ccagtacatg aactggctcg acagcggcaa cgccggcccc    1020
   tgcagcagca ccgagggcaa cccatccaac atcctggcca acaaccccaa cacgcacgtc    1080
   gtcttctcca acatccgctg gggagacatt gggtctacta cgaactcgac tgcgcccccg    1140
   cccccgcctg cgtccagcac gacgttttcg actacacgga ggagctcgac gacttcgagc    1200
   agcccgagct gcacgcagac tcactggggg cagtgcggtg gcattgggta cagcgggtgc    1260
   aagacgtgca cgtcgggcac tacgtgccag tatagcaacg actactactc gcaatgcctt    1320
   taa                                                                   1323
<210> 20
   <211> 440
   <212> PRT
   <213> Artificial Sequence
<220>
   <223> 309A1
<400> 20
   Gln Gln Pro Gly Ser Val Thr Pro Glu Val His Pro Lys Leu Pro Thr
   1               5                   10                  15
   Trp Gln Cys Thr Lys Ser Gly Gly Cys Val Glu Gln Asp Thr Ser Val
               20                  25                  30
   Val Leu Asp Trp Asn Tyr Arg Trp Phe His Thr Ser Asp Asn Thr Thr
           35                  40                  45
   Ser Cys Thr Thr Ser Ser Gly Ile Asp Ser Thr Leu Cys Pro Asp Ala
       50                  55                  60
   Ala Thr Cys Ala Glu Asn Cys Val Val Glu Gly Thr Asp Tyr Thr Ser
   65                  70                  75                  80
   Ser Gly Ile Glu Thr Ser Gly Ser Ser Leu Thr Leu Arg Gln Phe Val
                   85                  90                  95
   Lys Asp Ser Glu Gly Lys Thr Asn Ser Val Ser Pro Arg Ala Tyr Leu
               100                 105                 110
   Leu Gly Ala Asp Gly Asp Tyr Val Met Phe Lys Leu Leu Asn Gln Glu
           115                 120                 125
   Leu Ser Phe Asp Val Asp Val Ser Thr Leu Pro Cys Gly Glu Asn Ala
       130                 135                 140
   Ala Leu Tyr Phe Ser Glu Met Asp Lys Thr Gly Gly Arg Asn Glu His
   145                 150                 155                 160
   Asn Thr Gly Gly Ala Lys Tyr Gly Ser Gly Tyr Cys Asp Ala Gln Cys
                   165                 170                 175
   Pro Val Gln Thr Trp Asn Asn Gly Thr Leu Asn Thr Ser His Gln Gly
               180                 185                 190
   Ser Cys Cys Asn Glu Met Asp Ile Leu Glu Ala Asn Ser Lys Ala Glu
           195                 200                 205
   Ala Phe Thr Pro His Pro Cys Ile Gly Asp Asn Cys Asp Lys Gly Gly
       210                 215                 220
   Cys Gly Phe Asn Ser Tyr Ala Arg Gly Asn Lys Asn Tyr Trp Ala Pro
   225                 230                 235                 240
   Gly Gly Thr Leu Asp Thr Ser Lys Pro Phe Thr Met Val Thr Gln Phe
                   245                 250                 255
   Ile Thr Asp Asp Gly Thr Ser Ser Gly Lys Leu Ser Gln Ile Val Arg
               260                 265                 270
   Ser Tyr Val Gln Asn Gly Gln Lys Val Ala Ser Ala Val Ser Gly Gly
           275                 280                 285
   Asp Ser Ile Thr Val Glu Gly Cys Ser Ser Ala Ser Ala Tyr Gly Gly
       290                 295                 300
   Leu Ala Thr Met Gly Lys Ala Leu Ser Ser Gly Met Val Leu Val Phe
   305                 310                 315                 320
   Ser Ile Trp Asn Asp Asn Ser Gln Tyr Met Asn Trp Leu Asp Ser Gly
                   325                 330                 335
   Asn Ala Gly Pro Cys Ser Ser Thr Glu Gly Asn Pro Ser Asn Ile Leu
               340                 345                 350
   Ala Asn Asn Pro Asn Thr His Val Val Phe Ser Asn Ile Arg Trp Gly
           355                 360                 365
   Asp Ile Gly Ser Thr Thr Asn Ser Thr Ala Pro Pro Pro Pro Pro Ala
       370                 375                 380
   Ser Ser Thr Thr Phe Ser Thr Thr Arg Arg Ser Ser Thr Thr Ser Ser
   385                 390                 395                 400
   Ser Pro Ser Cys Thr Gln Thr His Trp Gly Gln Cys Gly Gly Ile Gly
                   405                 410                 415
   Tyr Ser Gly Cys Lys Thr Cys Thr Ser Gly Thr Thr Cys Gln Tyr Ser
               420                 425                 430
   Asn Asp Tyr Tyr Ser Gln Cys Leu
           435                 440
<210> 21
   <211> 1320
   <212> DNA
   <213> Artificial Sequence
<220>
   <223> 11G8
<400> 21
   cagcaaccgg gtaccagcac ccccgaggtc catcccaagt tgacaaccta caagtgtaca      60
   aagtccgggg ggtgcgtggc ccaggacacc tcggtggtcc ttgactggaa ctaccgctgg     120
   atgcacgacg caaactacaa ctcgtgcacc gtcaacggcg gcgtcaacac cacgctctgc     180
   cctgacgagg cgacctgtgg caagaactgc ttcatcgagg gcgtcgacta cgccgcctcg     240
   ggcgtcacga cctcgggcag cagcctcacc ctgaggcagt tcgtcaagga cagcgagggc     300
   aagaccaaca gcgtctctcc tcgggcctat ctcctgggcg ccgacggtga ctacgtgatg     360
   ttcaagctcc tcaaccagga gctgagcttc gacgtcgacg tctctacgct gccgtgtgga     420
   gagaacgcag cgctctactt ctctgagatg gacaagaccg ggggccgcaa cgaatataac     480
   acgggcggtg ccgagtacgg gagcggctac tgcgatgctc agtgccccgt ccagacatgg     540
   aacaacggca ccctcaacac tagccaccag ggctcgtgct gcaacgagat ggatatcctg     600
   gaggccaact cgaaggcgga agccttcacc cctcacccct gcatcggcga caactgcgac     660
   aagggcggtt gcggcttcaa ctcgtatgcg cgcggcaaca aaaactactg ggcgcccgga     720
   ggcaccctcg acacctccaa gcccttcacc atggtgaccc agttcatcac ggacgacggc     780
   accagctcgg gcaagcttag ccagatcgtg cgctcctacg tgcaaaacgg ccagaaggtc     840
   gccagcgccg tgtccggcgg cgacagcatc accgtcgagg gctgctcgtc ctccgacgcc     900
   tacggcggcc tcgtcggtat gggcgaggcc ctgggccgcg gcatggtgct cgtgttcagc     960
   atttggaacg acaacagcca gtacatgaac tggctcgaca gcggcaacgc cggcccctgc    1020
   agcagcaccg agggcaaccc atccaacatc ctggccaaca accccaacac gcacgtcgtc    1080
   ttctccaaca tccgctgggg agacattggg tctactacga actcgactgc gcccccgccc    1140
   ccgcctgcgt ccagcacgac gttttcgact acacggagga gctcgacgac ttcgagcagc    1200
   ccgagctgca cgcagactca ctgggggcag tgcggtggca ttgggtacag cgggtgcaag    1260
   acgtgcacgt cgggcactac gtgccagtat agcaacgact actactcgca atgcctttaa    1320
<210> 22
   <211> 439
   <212> PRT
   <213> Artificial Sequence
<220>
   <223> 11G8
<400> 22
   Gln Gln Pro Gly Thr Ser Thr Pro Glu Val His Pro Lys Leu Thr Thr
   1               5                   10                  15
   Tyr Lys Cys Thr Lys Ser Gly Gly Cys Val Ala Gln Asp Thr Ser Val
               20                  25                  30
   Val Leu Asp Trp Asn Tyr Arg Trp Met His Asp Ala Asn Tyr Asn Ser
           35                  40                  45
   Cys Thr Val Asn Gly Gly Val Asn Thr Thr Leu Cys Pro Asp Glu Ala
       50                  55                  60
   Thr Cys Gly Lys Asn Cys Phe Ile Glu Gly Val Asp Tyr Ala Ala Ser
   65                  70                  75                  80
   Gly Val Thr Thr Ser Gly Ser Ser Leu Thr Leu Arg Gln Phe Val Lys
                   85                  90                  95
   Asp Ser Glu Gly Lys Thr Asn Ser Val Ser Pro Arg Ala Tyr Leu Leu
               100                 105                 110
   Gly Ala Asp Gly Asp Tyr Val Met Phe Lys Leu Leu Asn Gln Glu Leu
           115                 120                 125
   Ser Phe Asp Val Asp Val Ser Thr Leu Pro Cys Gly Glu Asn Ala Ala
       130                 135                 140
   Leu Tyr Phe Ser Glu Met Asp Lys Thr Gly Gly Arg Asn Glu Tyr Asn
   145                 150                 155                 160
   Thr Gly Gly Ala Glu Tyr Gly Ser Gly Tyr Cys Asp Ala Gln Cys Pro
                   165                 170                 175
   Val Gln Thr Trp Asn Asn Gly Thr Leu Asn Thr Ser His Gln Gly Ser
               180                 185                 190
   Cys Cys Asn Glu Met Asp Ile Leu Glu Ala Asn Ser Lys Ala Glu Ala
           195                 200                 205
   Phe Thr Pro His Pro Cys Ile Gly Asp Asn Cys Asp Lys Gly Gly Cys
       210                 215                 220
   Gly Phe Asn Ser Tyr Ala Arg Gly Asn Lys Asn Tyr Trp Ala Pro Gly
   225                 230                 235                 240
   Gly Thr Leu Asp Thr Ser Lys Pro Phe Thr Met Val Thr Gln Phe Ile
                   245                 250                 255
   Thr Asp Asp Gly Thr Ser Ser Gly Lys Leu Ser Gln Ile Val Arg Ser
               260                 265                 270
   Tyr Val Gln Asn Gly Gln Lys Val Ala Ser Ala Val Ser Gly Gly Asp
           275                 280                 285
   Ser Ile Thr Val Glu Gly Cys Ser Ser Ser Asp Ala Tyr Gly Gly Leu
       290                 295                 300
   Val Gly Met Gly Glu Ala Leu Gly Arg Gly Met Val Leu Val Phe Ser
   305                 310                 315                 320
   Ile Trp Asn Asp Asn Ser Gln Tyr Met Asn Trp Leu Asp Ser Gly Asn
                   325                 330                 335
   Ala Gly Pro Cys Ser Ser Thr Glu Gly Asn Pro Ser Asn Ile Leu Ala
               340                 345                 350
   Asn Asn Pro Asn Thr His Val Val Phe Ser Asn Ile Arg Trp Gly Asp
           355                 360                 365
   Ile Gly Ser Thr Thr Asn Ser Thr Ala Pro Pro Pro Pro Pro Ala Ser
       370                 375                 380
   Ser Thr Thr Phe Ser Thr Thr Arg Arg Ser Ser Thr Thr Ser Ser Ser
   385                 390                 395                 400
   Pro Ser Cys Thr Gln Thr His Trp Gly Gln Cys Gly Gly Ile Gly Tyr
                   405                 410                 415
   Ser Gly Cys Lys Thr Cys Thr Ser Gly Thr Thr Cys Gln Tyr Ser Asn
               420                 425                 430
   Asp Tyr Tyr Ser Gln Cys Leu
           435
<210> 23
   <211> 1317
   <212> DNA
   <213> Artificial Sequence
<220>
   <223> 92A12
<400> 23
   cagcaaccgg gtaccagcac ccccgaggtc catcccaagt tgacaaccta caagtgtaca      60
   aagtccgggg ggtgcgtggc ccaggacacc tcggtggtcc ttgactggaa ctaccgctgg     120
   atgcacgacg caaactacaa ctcgtgcacc gtcaacggcg gcgtcaacac cacgctctgc     180
   cctgacgcgg cgacctgtgc gaagaactgc gtgatcgagc cggccaacta caccagcgcc     240
   ggcgtcacga cctcgggcga cagcctcacc atgtaccagt acgttcagag caacggcgtc     300
   tacaccaacg cctctcctcg ggcctatctc ctgggcgccg acggtgacta cgtgatgttc     360
   aagctcctca accaggagct gagcttcgac gtcgacgtct ctacgctgcc gtgtggagag     420
   aacgcagcgc tctacttctc tgagatggac aagaccgggg gccggaacga gcacaacacg     480
   ggcggtgcca agtacgggag cggctactgc gatgctcagt gccccgtcca gacatggaac     540
   aacggcaccc tcaacactag ccaccagggc tcgtgctgca acgagatgga tatcctggag     600
   gccaactcga aggcggaagc cttcacccct cacccctgca tcggcgacaa ctgcgacaag    660
   ggcggttgcg gcttcaactc gtatgcgcgc ggcaacaaaa actactgggc gcccggaggc     720
   accctcgaca cctccaagcc cttcaccatg gtgacccagt tcatcacgga cgacggcacc     780
   agctcgggca agcttagcca gatcgtgcgc tcctacgtgc aaaacggcca gaaggtcgcc     840
   agcgccgtgt ccggcggcga cagcatcacc gtcgagggct gctcgtcctc cgacgcctac     900
   ggcggcctcg tcggtatggg cgaggccctg ggccgcggca tggtgctcgt gttcagcatt     960
   tggaacgaca acagccagta catgaactgg ctcgacagcg gcaacgccgg cccctgcagc    1020
   agcaccgagg gcaacccatc caacatcctg gccaacaacc ccaacacgca cgtcgtcttc    1080
   tccaacatcc gctggggaga cattgggtct actacgaact cgactgcgcc cccgcccccg    1140
   cctgcgtcca gcacgacgtt ttcgactaca cggaggagct cgacgacttc gagcagcccg    1200
   agctgcacgc agactcactg ggggcagtgc ggtggcattg ggtacagcgg gtgcaagacg     1260
   tgcacgtcgg gcactacgtg ccagtatagc aacgactact actcgcaatg cctttaa       1317
<210> 24
   <211> 438
   <212> PRT
   <213> Artificial Sequence
<220>
   <223> 92A12
<400> 24
   Gln Gln Pro Gly Thr Ser Thr Pro Glu Val His Pro Lys Leu Thr Thr
   1               5                   10                  15
   Tyr Lys Cys Thr Lys Ser Gly Gly Cys Val Ala Gln Asp Thr Ser Val
               20                  25                  30
   Val Leu Asp Trp Asn Tyr Arg Trp Met His Asp Ala Asn Tyr Asn Ser
           35                  40                  45
   Cys Thr Val Asn Gly Gly Val Asn Thr Thr Leu Cys Pro Asp Ala Ala
       50                  55                  60
   Thr Cys Ala Lys Asn Cys Val Ile Glu Pro Ala Asn Tyr Thr Ser Ala
   65                  70                  75                  80
   Gly Val Thr Thr Ser Gly Asp Ser Leu Thr Met Tyr Gln Tyr Val Gln
                   85                  90                  95
   Ser Asn Gly Val Tyr Thr Asn Ala Ser Pro Arg Ala Tyr Leu Leu Gly
               100                 105                 110
   Ala Asp Gly Asp Tyr Val Met Phe Lys Leu Leu Asn Gln Glu Leu Ser
           115                 120                 125
   Phe Asp Val Asp Val Ser Thr Leu Pro Cys Gly Glu Asn Ala Ala Leu
       130                 135                 140
   Tyr Phe Ser Glu Met Asp Lys Thr Gly Gly Arg Asn Glu His Asn Thr
   145                 150                 155                 160
   Gly Gly Ala Lys Tyr Gly Ser Gly Tyr Cys Asp Ala Gln Cys Pro Val
                   165                 170                 175
   Gln Thr Trp Asn Asn Gly Thr Leu Asn Thr Ser His Gln Gly Ser Cys
               180                 185                 190
   Cys Asn Glu Met Asp Ile Leu Glu Ala Asn Ser Lys Ala Glu Ala Phe
           195                 200                 205
   Thr Pro His Pro Cys Ile Gly Asp Asn Cys Asp Lys Gly Gly Cys Gly
       210                 215                 220
   Phe Asn Ser Tyr Ala Arg Gly Asn Lys Asn Tyr Trp Ala Pro Gly Gly
   225                 230                 235                 240
   Thr Leu Asp Thr Ser Lys Pro Phe Thr Met Val Thr Gln Phe Ile Thr
                   245                 250                 255
   Asp Asp Gly Thr Ser Ser Gly Lys Leu Ser Gln Ile Val Arg Ser Tyr
               260                 265                 270
   Val Gln Asn Gly Gln Lys Val Ala Ser Ala Val Ser Gly Gly Asp Ser
           275                 280                 285
   Ile Thr Val Glu Gly Cys Ser Ser Ser Asp Ala Tyr Gly Gly Leu Val
       290                 295                 300
   Gly Met Gly Glu Ala Leu Gly Arg Gly Met Val Leu Val Phe Ser Ile
   305                 310                 315                 320
   Trp Asn Asp Asn Ser Gln Tyr Met Asn Trp Leu Asp Ser Gly Asn Ala
                   325                 330                 335
   Gly Pro Cys Ser Ser Thr Glu Gly Asn Pro Ser Asn Ile Leu Ala Asn
               340                 345                 350
   Asn Pro Asn Thr His Val Val Phe Ser Asn Ile Arg Trp Gly Asp Ile
           355                 360                 365
   Gly Ser Thr Thr Asn Ser Thr Ala Pro Pro Pro Pro Pro Ala Ser Ser
       370                 375                 380
   Thr Thr Phe Ser Thr Thr Arg Arg Ser Ser Thr Thr Ser Ser Ser Pro
   385                 390                 395                 400
   Ser Cys Thr Gln Thr His Trp Gly Gln Cys Gly Gly Ile Gly Tyr Ser
                   405                 410                 415
   Gly Cys Lys Thr Cys Thr Ser Gly Thr Thr Cys Gln Tyr Ser Asn Asp
               420                 425                 430
   Tyr Tyr Ser Gln Cys Leu
           435
<210> 25
   <211> 1320
   <212> DNA
   <213> Artificial Sequence
<220>
   <223> 240H12
<400> 25
   cagcaaccgg gtaccagcac ccccgaggtc catcccaagt tgacaaccta caagtgtaca      60
   actgctgggg ggtgcgtggc ccaggacacc tcggtggtcc ttgactgggg ctaccactgg     120
   attcacacgg tcgatgggta cacatcgtgc accacatcgt ccggcgtcga cagcacgctc     180
   tgccctgacg cggcgacctg tgcgaagaac tgcgtgatcg agccggccaa ctacaccagc     240
   gccggcgtca cgacctcggg cgacagcctc accatgtacc agtacgttca gagcaacggc     300
   gtctacacca acgcctctcc tcgggcctat ctcctgggcg ccgacggtga ctacgtgatg     360
   ttcaagctcc tcaaccagga gctgagcttc gacgtcgacg tctctacgct gccgtgtgga     420
   gagaacgcag cgctctactt ctctgagatg gacaagaccg ggggccggaa cgagcacaac     480
   acgggcggtg ccaagtacgg gagcggctac tgcgatgctc agtgccccgt ccagacatgg     540
   aacaacggca ccctcaacac tagccaccag ggctcgtgct gcaacgagat ggatatcctg     600
   gaggccaact cgaaggcgga agccttcacc cctcacccct gcatcggcga caactgcgac 660
   aagggcggtt gcggcttcaa ctcgtatgcg cgcggcaaca aaaactactg ggcgcccgga 720
   ggcaccctcg acacctccaa gcccttcacc atggtgaccc agttcatcac ggacgacggc 780
   accagctcgg gcaagcttag ccagatcgtg cgctcctacg tgcaaaacgg ccagaaggtc 840
   gccagcgccg tgtccggcgg cgacagcatc accgtcgagg gctgctcgtc cgcctcagcc 900
   tacggcggcc tcgccaccat gggcaaggcc ctgagcagcg gcatggtgct cgtgttcagc 960
   atttggaacg acaacagcca gtacatgaac tggctcgaca gcggcaacgc cggcccctgc 1020
   agcagcaccg agggcaaccc atccaacatc ctggccaaca accccaacac gcacgtcgtc 1080
   ttctccaaca tccgctgggg agacattggg tctactacga actcgactgc gcccccgccc 1140
   ccgcctgcgt ccagcacgac gttttcgact acacggagga gctcgacgac ttcgagcagc 1200
   ccgagctgca cgcagactca ctgggggcag tgcggtggca ttgggtacag cgggtgcaag 1260
   acgtgcacgt cgggcactac gtgccagtat agcaacgact actactcgca atgcctttaa 1320
<210> 26
   <211> 439
   <212> PRT
   <213> Artificial Sequence
<220>
   <223> 240H12
<400> 26
   Gln Gln Pro Gly Thr Ser Thr Pro Glu Val His Pro Lys Leu Thr Thr
   1 5 10 15
   Tyr Lys Cys Thr Thr Ala Gly Gly Cys Val Ala Gln Asp Thr Ser Val
   20 25 30
   Val Leu Asp Trp Gly Tyr His Trp Ile His Thr Val Asp Gly Tyr Thr
   35 40 45
   Ser Cys Thr Thr Ser Ser Gly Val Asp Ser Thr Leu Cys Pro Asp Ala
   50 55 60
   Ala Thr Cys Ala Lys Asn Cys Val Ile Glu Pro Ala Asn Tyr Thr Ser
   65 70 75 80
   Ala Gly Val Thr Thr Ser Gly Asp Ser Leu Thr Met Tyr Gln Tyr Val
   85 90 95
   Gln Ser Asn Gly Val Tyr Thr Asn Ala Ser Pro Arg Ala Tyr Leu Leu
   100 105 110
   Gly Ala Asp Gly Asp Tyr Val Met Phe Lys Leu Leu Asn Gln Glu Leu
           115                 120                 125
   Ser Phe Asp Val Asp Val Ser Thr Leu Pro Cys Gly Glu Asn Ala Ala
       130                 135                 140
   Leu Tyr Phe Ser Glu Met Asp Lys Thr Gly Gly Arg Asn Glu His Asn
   145                 150                 155                 160
   Thr Gly Gly Ala Lys Tyr Gly Ser Gly Tyr Cys Asp Ala Gln Cys Pro
                   165                 170                 175
   Val Gln Thr Trp Asn Asn Gly Thr Leu Asn Thr Ser His Gln Gly Ser
               180                 185                 190
   Cys Cys Asn Glu Met Asp Ile Leu Glu Ala Asn Ser Lys Ala Glu Ala
           195                 200                 205
   Phe Thr Pro His Pro Cys Ile Gly Asp Asn Cys Asp Lys Gly Gly Cys
       210                 215                 220
   Gly Phe Asn Ser Tyr Ala Arg Gly Asn Lys Asn Tyr Trp Ala Pro Gly
   225                 230                 235                 240
   Gly Thr Leu Asp Thr Ser Lys Pro Phe Thr Met Val Thr Gln Phe Ile
                   245                 250                 255
   Thr Asp Asp Gly Thr Ser Ser Gly Lys Leu Ser Gln Ile Val Arg Ser
               260                 265                 270
   Tyr Val Gln Asn Gly Gln Lys Val Ala Ser Ala Val Ser Gly Gly Asp
           275                 280                 285
   Ser Ile Thr Val Glu Gly Cys Ser Ser Ala Ser Ala Tyr Gly Gly Leu
       290                 295                 300
   Ala Thr Met Gly Lys Ala Leu Ser Ser Gly Met Val Leu Val Phe Ser
   305                 310                 315                 320
   Ile Trp Asn Asp Asn Ser Gln Tyr Met Asn Trp Leu Asp Ser Gly Asn
                   325                 330                 335
   Ala Gly Pro Cys Ser Ser Thr Glu Gly Asn Pro Ser Asn Ile Leu Ala
               340                 345                 350
   Asn Asn Pro Asn Thr His Val Val Phe Ser Asn Ile Arg Trp Gly Asp
           355                 360                 365
   Ile Gly Ser Thr Thr Asn Ser Thr Ala Pro Pro Pro Pro Pro Ala Ser
       370                 375                 380
   Ser Thr Thr Phe Ser Thr Thr Arg Arg Ser Ser Thr Thr Ser Ser Ser
   385                 390                 395                 400
   Pro Ser Cys Thr Gln Thr His Trp Gly Gln Cys Gly Gly Ile Gly Tyr
                   405                 410                 415
   Ser Gly Cys Lys Thr Cys Thr Ser Gly Thr Thr Cys Gln Tyr Ser Asn
               420                 425                 430
   Asp Tyr Tyr Ser Gln Cys Leu
           435
<210> 27
   <211> 1131
   <212> DNA
   <213> Chaetomium globosum
<400> 27
   cagcaaccgg gttcggtgac ccccgaggtc catcccaagt tgcccacctg gcagtgtaca      60
   aagtccgggg ggtgcgtgga gcaggacacc tcggtggtcc ttgactggaa ctaccgctgg     120
   ttccacacct cggacaacac cacctcgtgc accacgtcct cgggcatcga ctcgacgctc     180
   tgccctgacg cagcgacctg tgccgagaac tgcgtcgtgg agggcaccga ctacaccagc     240
   tcgggcatcg agacctcggg cagcagcctc accctgaggc agttcgtcaa ggacagcgag     300
   ggcaagacca acagcgtctc tcctcgggcc tatctcctgg gcgccgacgg tgactacgtg     360
   atgttcaagc tcctcaacca ggagctgagc ttcgacgtcg acgtctctac gctgccgtgt     420
   ggagagaacg cagcgctcta cttctctgag atggacaaga ccgggggccg gaacgagcac     480
   aacacgggcg gtgccaagta cgggagcggc tactgcgatg ctcagtgccc cgtccagaca     540
   tggaacaacg gcaccctcaa cactagccac cagggctcgt gctgcaacga gatggatatc     600
   ctggaggcca actcgaaggc ggaagccttc acccctcacc cctgcatcgg cgacaactgc     660
   gacaagggcg gttgcggctt caactcgtat gcgcgcggca acaaaaacta ctgggcgccc     720
   ggaggcaccc tcgacacctc caagcccttc accatggtga cccagttcat cacggacgac     780
   ggcaccagct cgggcaagct tagccagatc gtgcgctcct acgtgcaaaa cggccagaag     840
   gtcgccagcg ccgtgtccgg cggcgacagc atcaccgtcg agggctgctc gtcctccgac     900
   gcctacggcg gcctcgtcgg tatgggcgag gccctgggcc gcggcatggt gctcgccatg     960
   agcatttgga acgacgccag cggcttcatg aactggctcg acagcggcga caacggcccc    1020
   tgcaacgaga ccgagggcga cccagccaac atcctggcca accaccccga ttcgcaggtc    1080
   gtcctgtcca acatccgctg gggagacatt gactctactg ttcagctcta a 1131
<210> 28
   <211> 376
   <212> PRT
   <213> Chaetomium globosum
<400> 28
   Gln Gln Pro Gly Ser Val Thr Pro Glu Val His Pro Lys Leu Pro Thr
   1               5                   10                  15
   Trp Gln Cys Thr Lys Ser Gly Gly Cys Val Glu Gln Asp Thr Ser Val
               20                  25                  30
   Val Leu Asp Trp Asn Tyr Arg Trp Phe His Thr Ser Asp Asn Thr Thr
           35                  40                  45
   Ser Cys Thr Thr Ser Ser Gly Ile Asp Ser Thr Leu Cys Pro Asp Ala
       50                  55                  60
   Ala Thr Cys Ala Glu Asn Cys Val Val Glu Gly Thr Asp Tyr Thr Ser
   65                  70                  75                  80
   Ser Gly Ile Glu Thr Ser Gly Ser Ser Leu Thr Leu Arg Gln Phe Val
                   85                  90                  95
   Lys Asp Ser Glu Gly Lys Thr Asn Ser Val Ser Pro Arg Ala Tyr Leu
               100                 105                 110
   Leu Gly Ala Asp Gly Asp Tyr Val Met Phe Lys Leu Leu Asn Gln Glu
           115                 120                 125
   Leu Ser Phe Asp Val Asp Val Ser Thr Leu Pro Cys Gly Glu Asn Ala
       130                 135                 140
   Ala Leu Tyr Phe Ser Glu Met Asp Lys Thr Gly Gly Arg Asn Glu His
   145                 150                 155                 160
   Asn Thr Gly Gly Ala Lys Tyr Gly Ser Gly Tyr Cys Asp Ala Gln Cys
                   165                 170                 175
   Pro Val Gln Thr Trp Asn Asn Gly Thr Leu Asn Thr Ser His Gln Gly
               180                 185                 190
   Ser Cys Cys Asn Glu Met Asp Ile Leu Glu Ala Asn Ser Lys Ala Glu
           195                 200                 205
   Ala Phe Thr Pro His Pro Cys Ile Gly Asp Asn Cys Asp Lys Gly Gly
           210                 215                 220
   Cys Gly Phe Asn Ser Tyr Ala Arg Gly Asn Lys Asn Tyr Trp Ala Pro
   225                 230                 235                 240
   Gly Gly Thr Leu Asp Thr Ser Lys Pro Phe Thr Met Val Thr Gln Phe
                   245                 250                 255
   Ile Thr Asp Asp Gly Thr Ser Ser Gly Lys Leu Ser Gln Ile Val Arg
               260                 265                 270
   Ser Tyr Val Gln Asn Gly Gln Lys Val Ala Ser Ala Val Ser Gly Gly
           275                 280                 285
   Asp Ser Ile Thr Val Glu Gly Cys Ser Ser Ser Asp Ala Tyr Gly Gly
       290                 295                 300
   Leu Val Gly Met Gly Glu Ala Leu Gly Arg Gly Met Val Leu Ala Met
   305                 310                 315                 320
   Ser Ile Trp Asn Asp Ala Ser Gly Phe Met Asn Trp Leu Asp Ser Gly
                   325                 330                 335
   Asp Asn Gly Pro Cys Asn Glu Thr Glu Gly Asp Pro Ala Asn Ile Leu
               340                 345                 350
   Ala Asn His Pro Asp Ser Gln Val Val Leu Ser Asn Ile Arg Trp Gly
           355                 360                 365
   Asp Ile Asp Ser Thr Val Gln Leu
       370                 375
<210> 29
   <211> 1323
   <212> DNA
   <213> Aspergillus fumigatus
<400> 29
   cagcaaccgg ccgcgagttc tgctggtaac cccaagttga caacctacaa gtgtacaact      60
   gctggggggt gcgtggccca ggacacctcg gtggtccttg actggggcta ccactggatt     120
   cacacggtcg atgggtacac atcgtgcacc acatcgtccg gcgtcgacag cacgctctgc     180
   cctgacgcgg cgacctgtgc gaagaactgc gtgatcgagc cggccaacta caccagcgcc     240
   ggcgtcacga cctcgggcga cagcctcacc atgtaccagt acgttcagag caacggcgtc     300
   tacaccaacg cctctcctcg gctgtatctc ctgggccccg acaagaacta cgtgatgctg     360
   aagctcctag gccaggagct gaccttcgac gtcgacctct ctacactgcc gtgtggagag     420
   aacggcgccc tctacctgtc tgaaatgagc gccaccgggg gccgcaacga atataacacg     480
   ggcggtgccg agtacgggag cggctactgc gatgctcagt gccccgtcat cgcctggaag     540
   aacggcaccc tcaacactag cggcgcaagc tactgctgca acgagatgga tatcctggag     600
   gccaactcga gggcgaattc gtacacccct cacccctgca gtgccacgga ctgcgacaag    660
   ggcggttgcg gcttcaaccc ctatgctctc ggccaaaaaa gctactgggg ccccggaggc     720
   accgttgaca cctccaagcc cttcaccatc accacccagt tcatcacgaa cgacggcacc     780
   accaccggca ccctttccga aatccgccgc cagtacatgc aaaacggcaa ggtgatcgcc     840
   aatgccgttt cctccactgg cgtcaactcc atcaccgagg actggtgcac gtccgtcgac     900
   ggctcggccg ccacctttgg cggcctcacc accatgggca aggccctggg ccgcggcatg     960
   gtgctcatct tcagcatttg gaacgacgcc agcggcttta tgaactggct cgacagcggc    1020
   aacgccggcc cctgcagcag caccgagggc aacccagact tgatcaaggc ccagaacccc    1080
   acgacgcacg tcgtcttctc caacatccgc tggggagaca ttgggtctac tttcaagggt    1140
   tctgatggct cggtgacgac gacgacgtcg actacatcga ccaagaccac gacttcgacc    1200
   gcgccggggc caacgcagac tcactatggg cagtgcggtg gccaagggtg gactgggccc    1260
   acggcttgcg catcgcccta cacgtgccag gttctgaacc cgtggtactc gcaatgcctt    1320
   taa                                                                  1323
<210> 30
   <211> 440
   <212> PRT
   <213> Aspergillus fumigatus
<400> 30
   Gln Gln Pro Ala Ala Ser Ser Ala Gly Asn Pro Lys Leu Thr Thr Tyr
   1               5                   10                  15
   Lys Cys Thr Thr Ala Gly Gly Cys Val Ala Gln Asp Thr Ser Val Val
               20                  25                  30
   Leu Asp Trp Gly Tyr His Trp Ile His Thr Val Asp Gly Tyr Thr Ser
           35                  40                  45
   Cys Thr Thr Ser Ser Gly Val Asp Ser Thr Leu Cys Pro Asp Ala Ala
       50                  55                  60
   Thr Cys Ala Lys Asn Cys Val Ile Glu Pro Ala Asn Tyr Thr Ser Ala
   65                  70                  75                  80
   Gly Val Thr Thr Ser Gly Asp Ser Leu Thr Met Tyr Gln Tyr Val Gln
                   85                  90                  95
   Ser Asn Gly Val Tyr Thr Asn Ala Ser Pro Arg Leu Tyr Leu Leu Gly
               100                 105                 110
   Pro Asp Lys Asn Tyr Val Met Leu Lys Leu Leu Gly Gln Glu Leu Thr
           115                 120                 125
   Phe Asp Val Asp Leu Ser Thr Leu Pro Cys Gly Glu Asn Gly Ala Leu
       130                 135                 140
   Tyr Leu Ser Glu Met Ser Ala Thr Gly Gly Arg Asn Glu Tyr Asn Thr
   145                 150                 155                 160
   Gly Gly Ala Glu Tyr Gly Ser Gly Tyr Cys Asp Ala Gln Cys Pro Val
                   165                 170                 175
   Ile Ala Trp Lys Asn Gly Thr Leu Asn Thr Ser Gly Ala Ser Tyr Cys
               180                 185                 190
   Cys Asn Glu Met Asp Ile Leu Glu Ala Asn Ser Arg Ala Asn Ser Tyr
           195                 200                 205
   Thr Pro His Pro Cys Ser Ala Thr Asp Cys Asp Lys Gly Gly Cys Gly
       210                 215                 220
   Phe Asn Pro Tyr Ala Leu Gly Gln Lys Ser Tyr Trp Gly Pro Gly Gly
   225                 230                 235                 240
   Thr Val Asp Thr Ser Lys Pro Phe Thr Ile Thr Thr Gln Phe Ile Thr
                   245                 250                 255
   Asn Asp Gly Thr Thr Thr Gly Thr Leu Ser Glu Ile Arg Arg Gln Tyr
               260                 265                 270
   Met Gln Asn Gly Lys Val Ile Ala Asn Ala Val Ser Ser Thr Gly Val
           275                 280                 285
   Asn Ser Ile Thr Glu Asp Trp Cys Thr Ser Val Asp Gly Ser Ala Ala
       290                 295                 300
   Thr Phe Gly Gly Leu Thr Thr Met Gly Lys Ala Leu Gly Arg Gly Met
   305                 310                 315                 320
   Val Leu Ile Phe Ser Ile Trp Asn Asp Ala Ser Gly Phe Met Asn Trp
                   325                 330                 335
   Leu Asp Ser Gly Asn Ala Gly Pro Cys Ser Ser Thr Glu Gly Asn Pro
               340                 345                 350
   Asp Leu Ile Lys Ala Gln Asn Pro Thr Thr His Val Val Phe Ser Asn
           355                 360                 365
   Ile Arg Trp Gly Asp Ile Gly Ser Thr Phe Lys Gly Ser Asp Gly Ser
       370                 375                 380
   Val Thr Thr Thr Thr Ser Thr Thr Ser Thr Lys Thr Thr Thr Ser Thr
   385                 390                 395                 400
   Ala Pro Gly Pro Thr Gln Thr His Tyr Gly Gln Cys Gly Gly Gln Gly
                   405                 410                 415
   Trp Thr Gly Pro Thr Ala Cys Ala Ser Pro Tyr Thr Cys Gln Val Leu
               420                 425                 430
   Asn Pro Trp Tyr Ser Gln Cys Leu
           435                 440